# EUROPÄISCHE PATENTSCHRIFT

(11) **EP 2 055 714 B1**
(45) Veröffentlichungstag und Bekanntmachung des Hinweises auf die Patenterteilung: **07.05.2014**
(21) Anmeldenummer: 08016563.2
(22) Anmeldetag: 11.04.2003
(51) Int. Cl.: C07K 14/405, A61K 38/16

(54) **Biologische Photorezeptoren als direkt lichtgesteuerte Ionenkanäle und deren Verwendung**
Biological photo-receptors as directly light-controlled ion channels and their use
Photorécepteurs biologiques en tant que canaux ioniques commandés directement par la lumière et leur utilisation

(30) Priorität: 11.04.2002 DE 10216005
(43) Veröffentlichungstag der Anmeldung: 06.05.2009
(62) Teilanmeldung aus: 03727297.8
(73) Patentinhaber: Max-Planck-Gesellschaft zur Förderung der Wissenschaften e.V., 80539 München (DE)
(72) Erfinder: Hegemann, Peter, Prof. Dr., 14612 Falkensee (DE); Nagel, Georg, Prof. Dr., 97082 Würzburg (DE); Bamberg, Ernst, 65779 Kelkheim (DE)
(74) Vertreter: Wichmann, Hendrik

(56) Entgegenhaltungen:
- HEGEMANN PETER ET AL: "Algal sensory photoreceptors." JOURNAL OF PHYCOLOGY, Bd. 37, Nr. 5, Oktober 2001 (2001-10), Seiten 668-676, XP001154669 ISSN: 0022-3646
- DATABASE EMBL [Online] 2. Oktober 2001 (2001-10-02), "Chlamydomonas reinhardtii channelopsin-1 precursor, mRNA, complete cds." XP002533269 gefunden im EBI accession no. EMBL:AF385748 Database accession no. AF385748
- DATABASE WPI Section Ch, Week 200126 Thomson Scientific, London, GB; Class J04, AN 2001-247483 XP002254103 & JP 06 295350 A (SANYO ELECTRIC CO LTD) 21. Oktober 1994 (1994-10-21)
- PEROZO E ET AL: "VOLTAGE ACTIVATION OF RECONSTITUTED SODIUM CHANNELS: USE OF BACTERIORHODOPSIN AS A LIGHT-DRIVEN CURRENT SOURCE" BIOCHEMISTRY, AMERICAN CHEMICAL SOCIETY. EASTON, PA, US, Bd. 32, Nr. 39, 1993, Seiten 10471-10478, XP001015489 ISSN: 0006-2960
- BIESZKE JENNIFER A ET AL: "The nop-1 gene of Neurospora crassa encodes a seven transmembrane helix retinal-binding protein homologous to archaeal rhodopsins." PROCEEDINGS OF THE NATIONAL ACADEMY OF SCIENCES OF THE UNITED STATES, Bd. 96, Nr. 14, 6. Juli 1999 (1999-07-06), Seiten 8034-8039, XP002254100 July 6, 1999 ISSN: 0027-8424
- NAGEL GEORG ET AL: "Channelrhodopsin-1: a light-gated proton channel in green algae." SCIENCE. UNITED STATES 28 JUN 2002, Bd. 296, Nr. 5577, 28. Juni 2002 (2002-06-28), Seiten 2395-2398, XP002254101 ISSN: 1095-9203
- SINESHCHEKOV OLEG A ET AL: "Two rhodopsins mediate phototaxis to low- and high-intensity light in Chlamydomonas reinhardtii." PROCEEDINGS OF THE NATIONAL ACADEMY OF SCIENCES OF THE UNITED STATES, Bd. 99, Nr. 13, 25. Juni 2002 (2002-06-25), Seiten 8689-8694, XP002254102 http://www.pnas.org June 25, 2002 ISSN: 0027-8424
- DATABASE EMBL [Online] 26 März 2002 'Chlamydomonas reinhardtii acop2 mRNA for archaeal-type opsin 2, complete cds.' Retrieved from EBI, accession no. EM_PL:AB058891 Database accession no. AB058891

## Beschreibung

Die vorliegende Erfindung betrifft biologische Photorezeptoren, insbesondere als di-rekt lichtgesteuerte Ionenkanäle und DNA, die diese Kodiert, wie in den Ansprüchen definiert.

Es ist seit längerem bekannt, dass eine Vielzahl zentraler Vorgänge in pflanzlichen und tierischen Zellen ganz oder teilweise über Änderungen der intrazellulären Konzentration bestimmter Ionen, beispielsweise der Protonenkonzentration, und Änderungen des Membranpotentials und der Ionengradienten über die Membran gesteuert wird. Dementsprechend ist die Aufklärung von Mechanismen der intrazellulären lonen-Regulation, insbesondere der pH-Regulation, und der Mechanismen von spannungsabhängigen Ionenkanälen und lonentransportern Gegenstand ausgedehnter Forschungsaktivitäten. Für solche Forschungen ist eine schnelle und leichte Messung und/oder Manipulation von intrazellulären lonenkonzentrationen, insbesondere Protonenkonzentrationen, sowie von elektrischen Gradienten und lonengradienten, insbesondere Protonengradienten, über Zellmembranen von großem Interesse.

Ein grundsätzlich sehr vorteilhafter Weg zur Ermöglichung solcher Messungen und/oder Manipulationen wäre die Einführung eines lichtgesteuerten Ionenkanals in eine solche Membran, um gezielt das Membranpotential und/oder den Fluss bestimmter Ionen, z.B. Protonen, durch die Membran zu verändern.

Im Stand der Technik sind eine Reihe lichtgesteuerter lonentransportsysteme bekannt. Man unterscheidet dabei zwischen passiven und aktiven lonentransportsystemen. Bei den passiven lonentransportsystemen stehen die eigentlichen Transportmoleküle, die so genannten Ionenkanäle, in Wechselwirkung mit separaten Photorezeptor-Komplexen und werden indirekt über diese Photorezeptoren gesteuert. Beispiele für solche mit einem Ionenkanal wechselwirkenden Photorezeptor-Komplexe sind die G-Protein aktivierenden Rhodopsine; siehe z.B. Müller, F., Kaupp, U. B., Signaltransduktion in Sehzellen, Naturwissenschaften 85 (1998) 49-6. Da diese bekannten passiven lonentransportsysteme das Zusammenwirken mehrerer Proteine und anderer Komponenten erfordern, ist es problematisch, sie unter Beibehaltung ihrer Funktion, z.B. durch Co-Expression der entsprechenden Proteine in rekombinanten Systemen, in andere Zellen einzuführen.

Beispiele für lichtgesteuerte aktive lonentransportmoleküle in einer Zellmembran sind die Rhodopsine aus Archaeen (Archaebakterien). Ein bekannter Vertreter eines Archaeen-Rhodopsins ist das Bakteriorhodopsin, bestehend aus einer Proteinkomponente, dem Bakterioopsin, und einem über eine Schiffsche Base gebundenen Retinal. Nach Anregung durch Licht geeigneter Wellenlänge transportiert Bakteriorhodopsin Protonen durch die Membran aus dem Zellinneren nach außen. Der lonentransport verläuft in der Regel langsam (< 100 H⁺/ Sekunde) und die Transportrichtung des Bakteriorhodopsins kann nicht frei bestimmt werden. *In vivo* befördert Bakteriorhodopsin Protonen auch gegen einen bestehenden elektrochemischen Gradienten, der sich aus einem pH-Gradienten und einem elektrischem Gradienten ΔΨ zusammensetzt. Eine solche lichtgesteuerte Protonenpumpe stellt ein vergleichsweise einfaches System dar. Bakteriorhodopsin und verwandte Retinalbindende Proteine wie die Chloridpumpe Halorhodopsin wurden bereits heterolog exprimiert und waren im heterogenen Wirt zum lichtgetriebenen aktiven Membrantransport von Ionen, z. B. H⁺ oder Cl⁻, in der Lage.

Für viele Zwecke sind die bekannten Systeme aufgrund ihrer Komplexität bzw. der vorgegebenen Transportrichtung jedoch ungeeignet.

Eine Aufgabe der vorliegenden Erfindung ist, Mittel und Wege bereitzustellen, die eine schnelle und leichte Messung und/oder Manipulation von intrazellulären lonenkonzentrationen, insbesondere Protonenkonzentrationen, sowie von elektrischen Gradienten über eine Zellmembran erlaubt.

Eine weitere Aufgabe ist, ein System, das für das Hochdurchsatzscreening von biologischen Molekülen, insbesondere von pH-regulatorischen Membranproteinen und spannungsabhängigen Membranproteinen geeignet ist, bereitzustellen.

Die der Erfindung zu Grunde liegenden Aufgaben werden erfindungsgemäß durch einen biologischen Photorezeptor gemäß dem unabhängigen Anspruch 1 und 8 gelöst, und zwar insbesondere als direkt lichtgesteuerter lonenkanal zur Veränderung der lonenleitfähigkeit einer Membran mit Hilfe von Licht. Vorteilhafte Weiterbildungen sind Gegenstand der jeweiligen abhängigen Ansprüche.

Die vorliegende Erfindung schafft einen biologischen Photorezeptor, insbesondere als direkt lichtgesteuerter Ionenkanal zur Veränderung der lonenleitfähigkeit einer Membran mit Hilfe von Licht, bei welchem der Photorezeptor ein Apoprotein und ein lichtsensitives Polyen aufweist, wobei das lichtsensitive Polyen kovalent an das Apoprotein gebunden ist, mit dem Apoprotein wechselwirkt und als lichtempfindliches Tor fungiert, und bei welchem das Apoprotein ein Ionenkanal bildendes Opsin oder ein Derivat oder Fragment eines Ionenkanal bildenden Opsins ist, wie in den Ansprüchen definiert.

Die vorliegende Erfindung löst die Aufgaben also u.a. durch die Schaffung und Verwendung einer neuen Klasse von biologischen Photorezeptoren mit bisher unbekannter Funktion, die direkt lichtgesteuerte passive lonentransportsysteme (einen lichtgesteuerten Ionenkanal) darstellen. Dieser Photorezeptor umfasst ein Apoprotein, das eine lonenleitfähigkeit vermittelt, und ein kovalent an das Apoprotein gebundenes lichtsensitives Polyen, das mit dem Apoprotein wechselwirkt und als lichtempfindliches Tor fungiert. Überraschenderweise ist es erfindungsgemäß gelungen, die Vorteile eines relativ einfachen direkt lichtgesteuerten Systems mit den Vorteilen eines passiven Ionenkanals zu verbinden.

Bei dem Apoprotein handelt es sich um ein Membranprotein mit mindestens 5 Transmembranhelices, das zur Bindung eines lichtsensitiven Polyens fähig ist. Bevorzugt sind Transmembranproteine mit sechs oder sieben Transmembranhelices. Transmembranproteine mit mehr als sieben Helices, beispielsweise acht, neun oder zehn Transmembranhelices, sind von der Erfindung jedoch ebenfalls umfasst. Darüber hinaus umfasst die Erfindung Transmembranproteine, die zusätzlich zu dem Transmembrananteil C- und/oder N-terminale Sequenzen enthalten, wobei die C-terminalen Sequenzen in das Innere des von der Membran umschlossenen Lumens, beispielsweise das Cytoplasma einer Zelle oder das Innere eines Liposomes, hineinreichen können, oder auch an der Membranaußenoberfläche angeordnet sein können. Das gleiche gilt für die gegebenenfalls vorhandenen N-terminalen Sequenzen, die ebenfalls sowohl intraluminal angeordnet sein können als auch auf der äußeren Oberfläche der Membran. Die Länge der C- und/oder N-terminalen Sequenzen unterliegt im Prinzip keiner Beschränkung; bevorzugt sind jedoch Apoproteine mit C-terminalen, nicht in die Membran eingebetteten Sequenzen mit 1 bis 1000 Aminosäuren, bevorzugt 1 bis 500, besonders bevorzugt 5 bis 50 Aminosäuren. Unabhängig von der Länge der C-terminalen Sequenzen umfassen die N-terminal angeordneten, nicht in die Membran eingebetteten Sequenzen bevorzugt 1 bis 500 Aminosäuren, besonders bevorzugt 5 bis 50 Aminosäuren.

Der Begriff der Transmembranhelix ist dem Fachmann bekannt. Generell handelt es sich um α-helicale Proteinstrukturen, die in der Regel 20 bis 25 Aminosäuren umfassen. Je nach Beschaffenheit der Membran, bei der es sich um eine natürliche Membran, beispielsweise eine Zell- oder Plasmamembran, oder auch eine synthetische Membran handeln kann, können die Transmembransegmente jedoch auch kürzer oder länger sein. Beispielsweise können Transmembransegmente in artifiziellen Membranen bis zu 30 Aminosäuren umfassen, andererseits aber auch nur wenige Aminosäuren, zum Beispiel 12 bis 16.

Der erfindungsgemäße Ionenkanal kann prinzipiell dem passiven Transport aller physiologisch wichtigen Ionen dienen. Die bekanntesten lonentransportsysteme transportieren Na⁺, K⁺, Ca²⁺, H⁺ oder Cl⁻. In einer bevorzugten Ausführungsform ist der erfindungsgemäße Ionenkanal ein Protonenkanal.

In einer besonders bevorzugten Ausführungsform umfasst der Protonenkanal als Apoproteinanteil ein Opsin-Protein oder ein Derivat oder Fragment eines natürlich vorkommenden Opsinproteins. Als Opsine werden hier Apoproteine bezeichnet, die kovalent mit einem Retinoid-Chromophor verbunden sind und lonenleitfähigkeit aufweisen, sobald sie Licht absorbieren. Ein Molekül, das ein kovalent mit einem Opsin verbundenes Retinoid-Chromophor enthält, wird als Rhodopsin bezeichnet.

Ein Derivat eines natürlich vorkommenden und als lichtgeschalteter Ionenkanal fungierenden Opsinmoleküles ist dem Original gegenüber durch einen Austausch einer oder mehrerer Aminosäuren, durch eine Insertion und/oder eine Deletion einer oder mehrerer Aminosäuren an einer oder mehreren Positionen verändert. Sowohl die natürlich vorkommenden Opsinmoleküle als auch ihre Derivate sollten im Bereich der 5 Transmembranhelices, die den Helices 3 bis 7 im Bakteriorhodopsin entsprechen, eine Identität von mindestens 15 % mit der Sequenz des Bakteriorhodopsins aufweisen. Eine Identität von 20 % oder mehr zwischen dem Derivat eines Kanal-Opsins und Bakteriorhodopsin, bezogen nur auf den Bereich der Helices 3 bis 7 in Bakteriorhodopsin, ist dabei bevorzugt. Die Identität in den Bereichen des Opsinderivates, die nicht den Helices 3 bis 7 entsprechen, kann dagegen weitaus geringer sein.

Mit dem Begriff "Identität" wird dabei der Grad der Verwandtschaft zwischen zwei oder mehr Proteinsequenzen bezeichnet, der durch die Übereinstimmung zwischen diesen Sequenzen bestimmt wird. Der Prozentsatz der Identität ergibt sich aus dem Prozentsatz identischer Aminosäuren in zwei oder mehr Sequenzen unter Berücksichtigung von Lücken und anderen Sequenzbesonderheiten.

Die Identität miteinander verwandter Protein-Moleküle kann mit Hilfe bekannter Verfahren bestimmt werden. In der Regel werden spezielle Computerprogramme mit den besonderen Anforderungen Rechnung tragenden Algorithmen eingesetzt. Bevorzugte Verfahren zur Bestimmung der Identität erzeugen zunächst die größte Übereinstimmung zwischen den untersuchten Sequenzen. Computerprogramme zur Bestimmung der Identität zwischen zwei Sequenzen umfassen, sind jedoch nicht eingeschränkt auf, das GCG-Programmpaket, einschließlich GAP (Devereux et al., 1984); Genetics Computer Group University of Wisconsin, Madison, (WI)); BLASTP, BLASTN und FASTA (Altschul et al., NCB NLM NIH Bethesda MD 20894; Altschul et al., 1990). Auch der bekannte Smith Waterman-Algorithmus kann zur Bestimmung von Identität verwendet werden.

Bevorzugte Parameter für den Sequenz-Vergleich umfassen die nachstehenden:
Algorithmus: Altschul et al., 1990 (Basic local alignment search tool, J. Mol. Biol. 215, 403-410) (=BLAST)
Vergleichsmatrix: BLOSUM 62 (Henikoff and Henikoff, 1992, Amino acid substitutions from protein blocks. PNAS 89, 10915-10919)
Übereinstimmung (matches) = variabel
Nichtübereinstimmung (mismatch) = variabel
Lücken-Wert: open 10
Lückenlängen-Wert (gap length penalty): 1

Das GAP-Programm ist auch zur Verwendung mit den vorstehenden Parametern geeignet. Die vorstehenden Parameter sind die Standardparameter (default parameters) für Protein-Vergleiche.

Weitere beispielhafte Algorithmen, Lückenöffnungs-Werte (gap opening penalties), Lückenausdehnungs-Werte (gap extension penalties), Vergleichsmatrizen einschließlich der im Programm-Handbuch, Wisconsin-Paket, Version 9, September 1997, genannten, können verwendet werden. Die Auswahl wird von dem durchzuführenden Vergleich abhängen und weiterhin davon, ob der Vergleich zwischen Sequenzpaaren, wobei GAP oder Best Fit bevorzugt sind, oder zwischen einer Sequenz und einer umfangreichen Sequenz-Datenbank, wobei FASTA oder BLAST bevorzugt sind, durchgeführt wird.

Für die aktive Protonenpumpe Bakteriorhodopsin ist bekannt, dass Asp(D)⁹⁶ eine für die Protonenpumpfunktion essentielle Aminosäure ist. Weiter sind die folgenden 16 Aminosäuren des Bakteriorhodopsins am Protonennetzwerk beteiligt:
F⁴², T⁴⁶, Y⁵⁷, R⁸², D⁸⁵, T⁸⁹, L⁹³, T¹⁰⁷, W¹⁸², Y¹⁸⁵, W¹⁸⁹, E¹⁹⁴, E²⁰⁴, D²¹², K²¹⁶, F²¹⁹

Im erfindungsgemäßen Ionenkanal ist an der dem D⁹⁶ der Bakteriorhodopsinsequenz entsprechenden Position eine andere Aminosäure als "D". E²⁰⁴ ist vorzugsweise ausgetauscht durch S. In einer Ausführungsform sind von den weiteren 15 Aminosäuren jedoch mindestens 8 identisch erhalten oder lediglich durch konservativen Austausch verändert. Bei den Aminosäuren, die möglichst identisch erhalten sein sollten, handelt es sich bevorzugt um T⁴⁶, Y⁵⁷, R⁸², T⁸⁹, T¹⁰⁷, W¹⁸², E¹⁹⁴, D²¹² und K²¹⁶. Konservativ ausgetauschte Aminosäuren sind bevorzugt F⁴², D⁸⁵, L⁹³, Y¹⁸⁵, W¹⁸⁹ und F²¹⁹ Der Fachmann weiß dabei, dass für einen konservativen Austausch eine Aminosäure ausgewählt wird, die der auszutauschenden Aminosäure funktionell ähnlich ist. Dabei werden Austausche normalerweise innerhalb der folgenden Gruppen vorgenommen:

| | | | | | |
|---|---|---|---|---|---|
| (a) | Ala (A) | Ser (S) | Thr (T) | Pro (P) | Gly (G); |
| (b) | Asn (N) | Asp (D) | Glu (E) | Gln (Q); | |
| (c) | His (H) | Arg (R) | Lys (K) | | |
| (d) | Met (M) | Leu (L) | Ile (I) | Val (V) | und |
| (e) | Phe (F) | Tyr (Y) | Trp (W). | | |

Bezogen auf die oben angegebenen Aminosäuren, sind bevorzugte Austausche die folgenden: F42Y, D85E, Y185F, W189F und F219W.

In einer weiteren bevorzugten Ausführungsform sind ein oder mehrere weitere der folgenden Positionen, bezogen auf Bakteriorhodopsin, im erfindungsgemäßen lonenkanal erhalten: Y⁸³, W⁸⁶, P⁹¹, G¹²², P¹⁸⁶ und G¹⁹⁵.

In einer weiteren bevorzugten Ausführungsform enthält ein erfindungsgemäßer passiver Protonenkanal ein Apoprotein mit der Konsensussequenz L(I)DxxxKxxW(F,Y). In Klammern angegebene Aminosäuren können jeweils die vorhergehende Aminosäure ersetzen. Bei dieser Konsensussequenz handelt es sich um das die Retinalbindende Aminosäure Lysin umgebende Motiv. Im Bakteriorhodopsin entspricht das "K" an Position 6 der Konsensussequenz dem K²¹⁶ in der 7. Helix des Bakteriorhodopsins.

In einer bevorzugten Ausführungsform umfasst der Ionenkanal ein Apoprotein aus niederen Eukaryonten.

Besonders bevorzugt sind dabei motile Grünalgen, insbesondere Chlorophyceen. Apoproteine aus Volvocales sind dabei besonders interessant. In der am meisten bevorzugten Ausführungsform ist das Apoprotein ein Opsinprotein aus *Chlamydomonas reinhardtii.*

Es wurden aus *Chlamydomonas reinhardtii* zwei verschiedene Proteine mit bekannter Sequenz funktionell exprimiert und erstmals als passive Ionentransportsysteme identifiziert. Dabei handelt es sich um das Channelopsin1 (= CHOP-1; auch Chlamyopsin-3 = COP3 genannt) und das Channelopsin2 (= CHOP-2; auch Chlamyopsin-4 = COP4 genannt) (AB 05 8891).

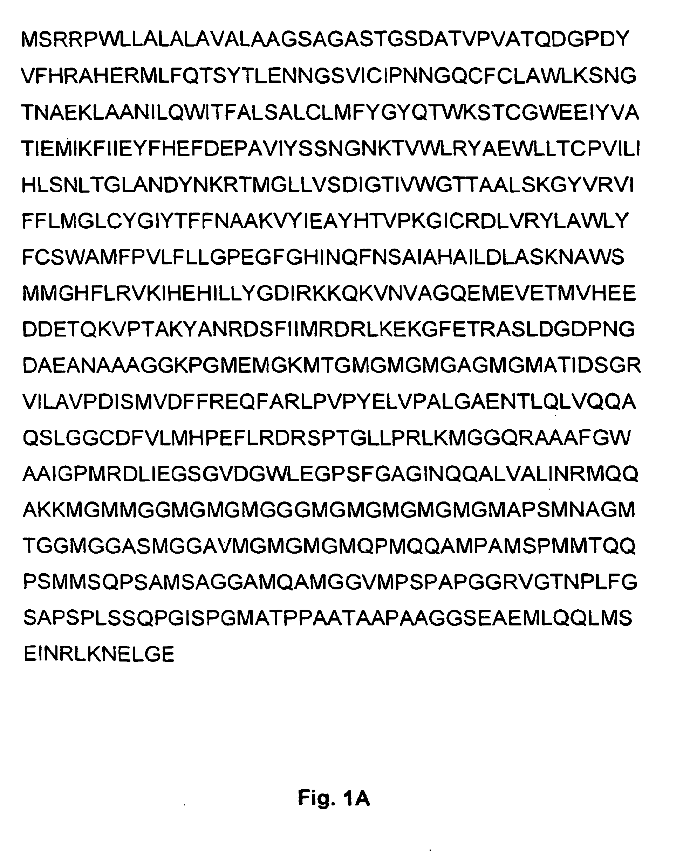

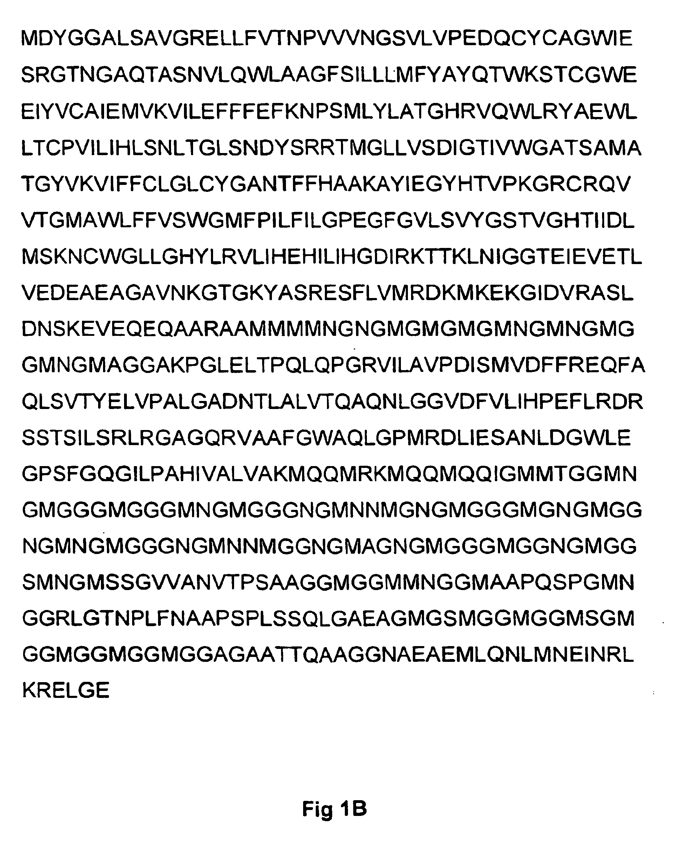

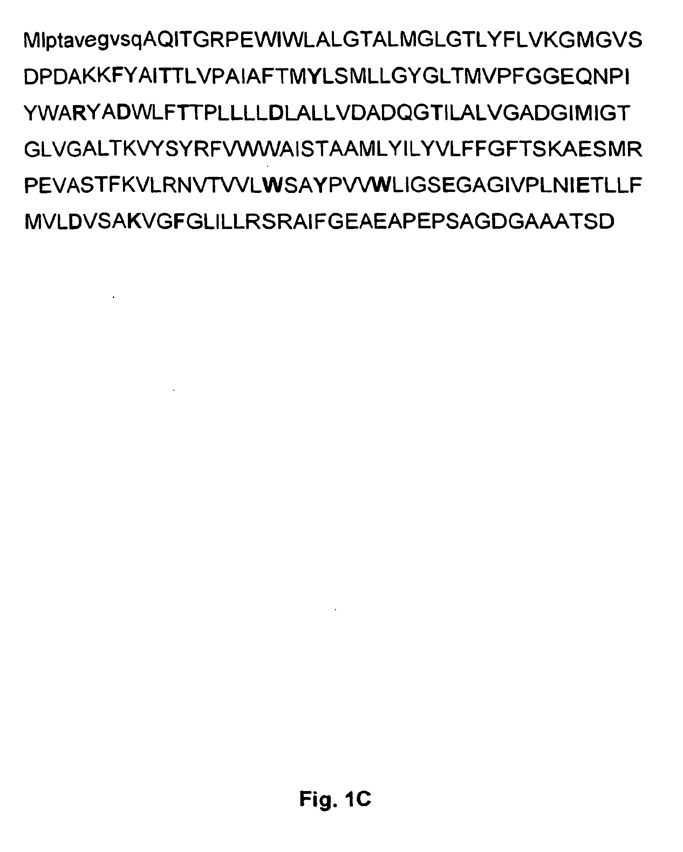

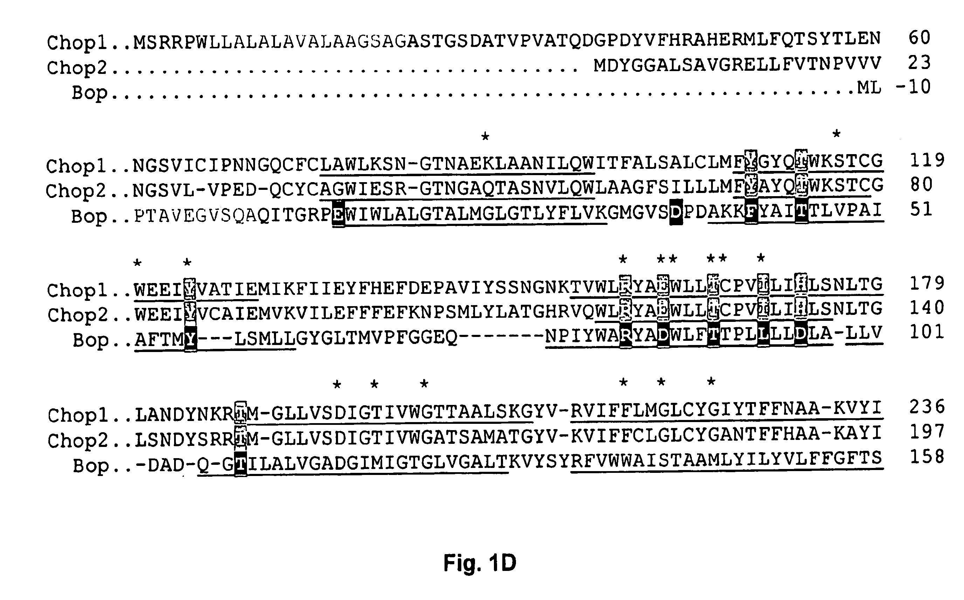

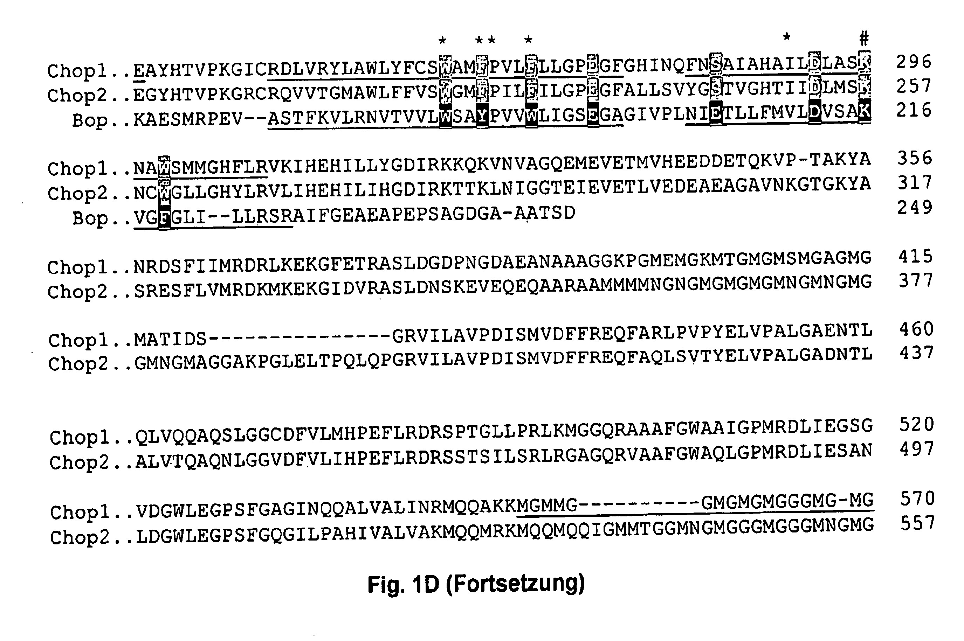

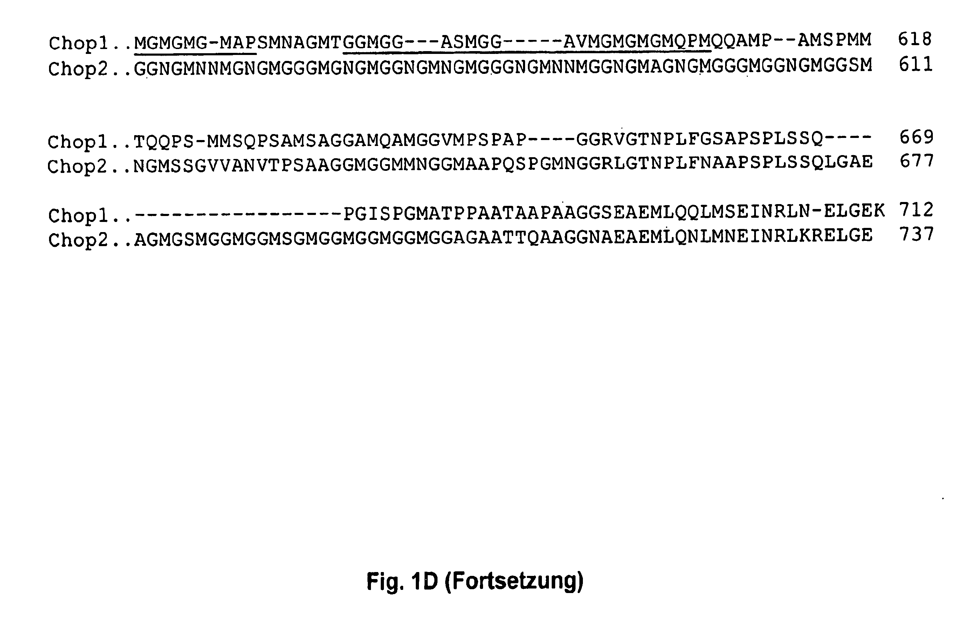

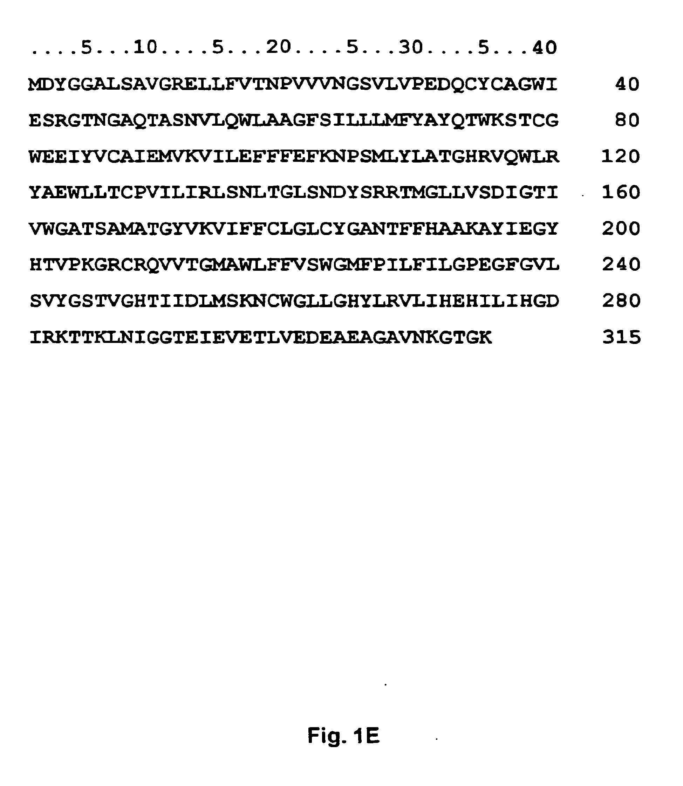

Das CHOP-1-Protein weist ein Molekulargewicht von 76 kD und eine Länge von 712 Aminosäuren auf. Es wurde anhand von überlappenden partiellen cDNA-Sequenzen in einer *C. reinhardtii*-EST-Datenbank (Asamizu et al., DNA Research 7, 305-7 (2000)) identifiziert. Seine Aminosäuresequenz ist in Fig. 1 der vorliegenden Anmeldung dargestellt. Das Core-Protein (Aminosäuren 76-309) umfasst 7 hypothetische Transmembran-Segmente mit 15-20% Homologie zu den sensorischen Archaeen-Rhodopsinen, den lonentransportern Bakteriorhodopsin (BR) und Halorhodopsin (HR), sowie zu einem erst kürzlich identifizierten Rhodopsin aus dem Pilz *Neurospora crassa* (NOP1). Diese Homologiegrade sind zwar quantitativ relativ gering, es sind jedoch im Vergleich zu BR speziell diejenigen Aminosäuren konserviert, welche die Retinal-Bindungsstelle und das H⁺-Transport-Netzwerk in BR definieren. Das beobachtete Konsensus-Motiv LDxxxKxxW legt nahe, dass im CHOP-1 K²⁹⁵ die Retinalbindende Aminosäure ist. 9 von 22 Aminosäuren, welche im Bakteriorhodopsin in direktem Kontakt mit dem Retinal stehen, sind in CHOP-1 identisch erhalten und 4 weitere reflektieren lediglich konservative Änderungen ((Fig.1); Nagel et al., in Vorbereitung).

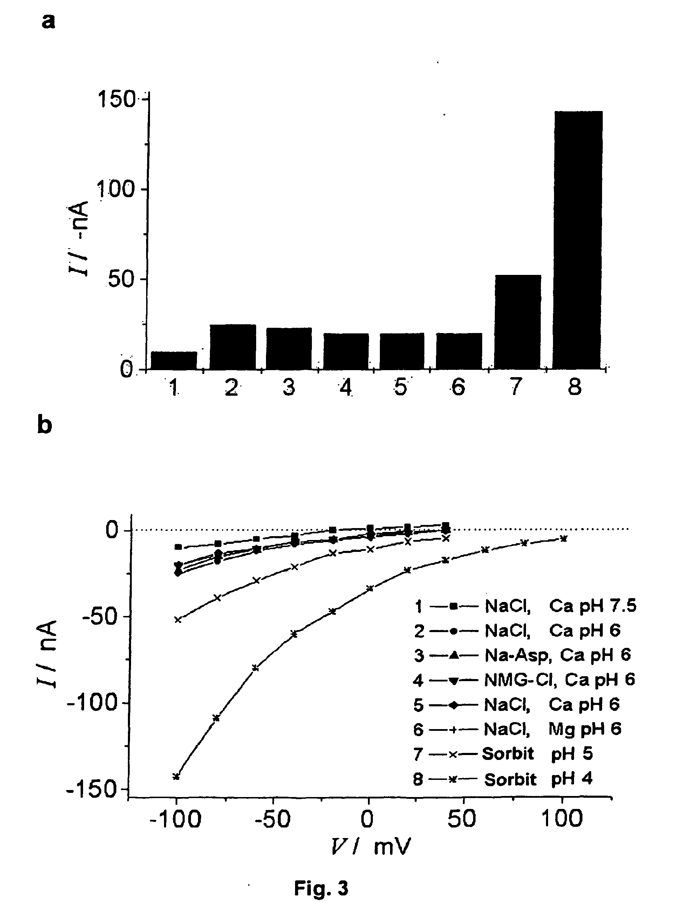

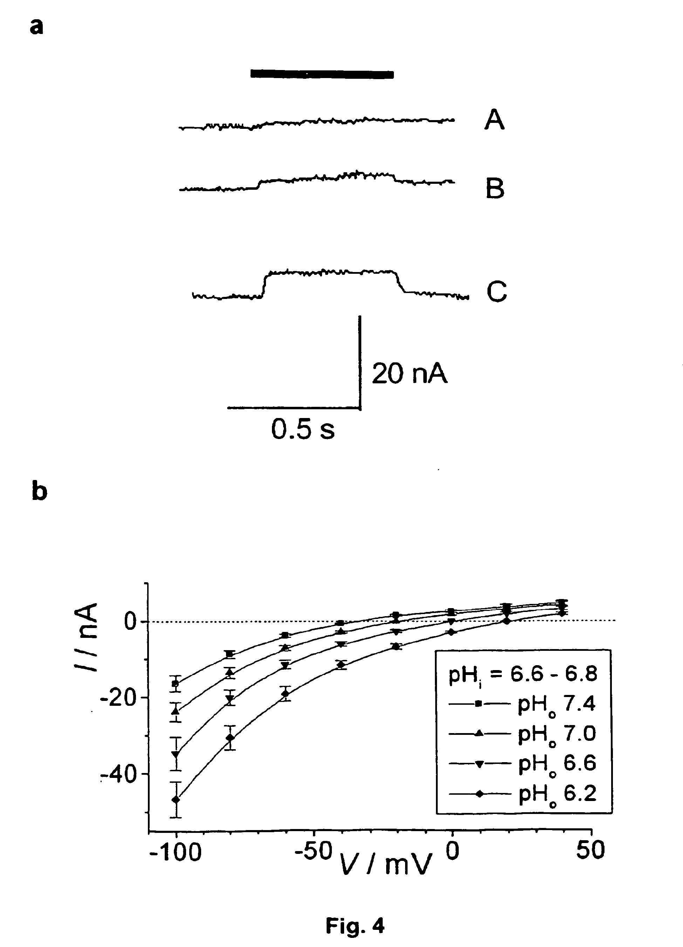

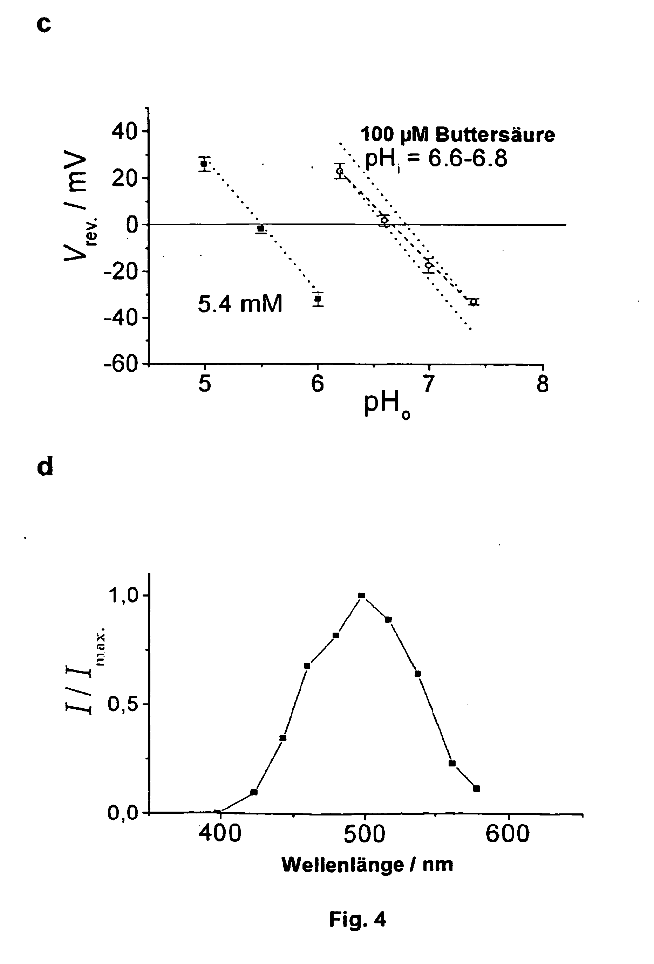

Eingehende Untersuchungen der lichtgesteuerten lonentransportfunktion des CHOP-1-Proteins in Oozyten von *Xenopus laevis* zeigten, dass es sich bei den transportierten Ionen um Protonen handelt (Fig. 3), außerdem, dass der lonentransport rein passiver Natur ist (Fig. 4a-c). Der induzierte Photostrom und damit der Ionentransport ist von der Wellenlänge des Anregungslichtes abhängig und erreicht ein Maximum bei 500 nm (Fig. 4d).

Analoge Versuche mit zwei kürzeren Fragmenten des CHOP-1-Proteins, welche die Aminosäuren 1-346 bzw. 1-517 umfassten, lieferten Ergebnisse, welche mit denjenigen des CHOP-1-Proteins vollständiger Länge im Wesentlichen identisch waren. Dies demonstriert, dass ein großer Teil des carboxyterminalen Bereichs des CHOP-1-Proteins für die lonentransportfunktion nicht erforderlich ist.

Das CHOP-1-Protein aus *C. reinhardfii* stellt den ersten identifizierten Vertreter eines neuen direkt lichtgesteuerten passiven lonentransportproteins dar. Strukturell und/oder funktionell ähnliche Rhodopsin-Proteine kommen auch in anderen Mikroalgen sowie in Gameten und Zoosporen von Makroalgen und möglicherweise auch in anderen Organismen vor.

Das zweite als Apoprotein eines lichtgeschalteten Ionenkanals identifizierte Protein ist Channelopsin2 (CHOP-2), dessen 737 Aminosäuren umfassende Sequenz ebenfalls in Figur 1 gezeigt ist. Es zeigt eine Homologie von 52.7 % zu CHOP-1. Die über die Homologie zwischen BR und CHOP-1 sowie Modellberechnungen identifizierten, für den Transport wichtigen Aminosäuren sind auch in CHOP-2 weitgehend konserviert. Auch für dieses Rhodopsin wurde eine lichtgeschaltete passive Protonenleitfähigkeit durch Expression in Xenopus-Oocyten erstmalig nachgewiesen. Der mit CHOP-2 als Apoprotein gebildete Ionenkanal unterscheidet sich von dem mit CHOP-1 gebildeten hinsichtlich seiner Einheitsleitfähigkeit, seiner Inaktivierung im Dauerlicht und der Form der Strom-Spannungskurve. Channelrhodopsin-2 (ChR2), welches sich aus dem Protein Channelopsin-2 (CHOP-2) und Retinal zusammensetzt, ist ein lichtgesteuerter Kationenkanal, der z.B. für Li⁺, Na⁺, K⁺, Ba²⁺, Sr⁺ und Ca²⁺, nicht jedoch für Mg²⁺, permeabel ist. Das Maximum des Anregungslichts liegt bei 440-480 nm.

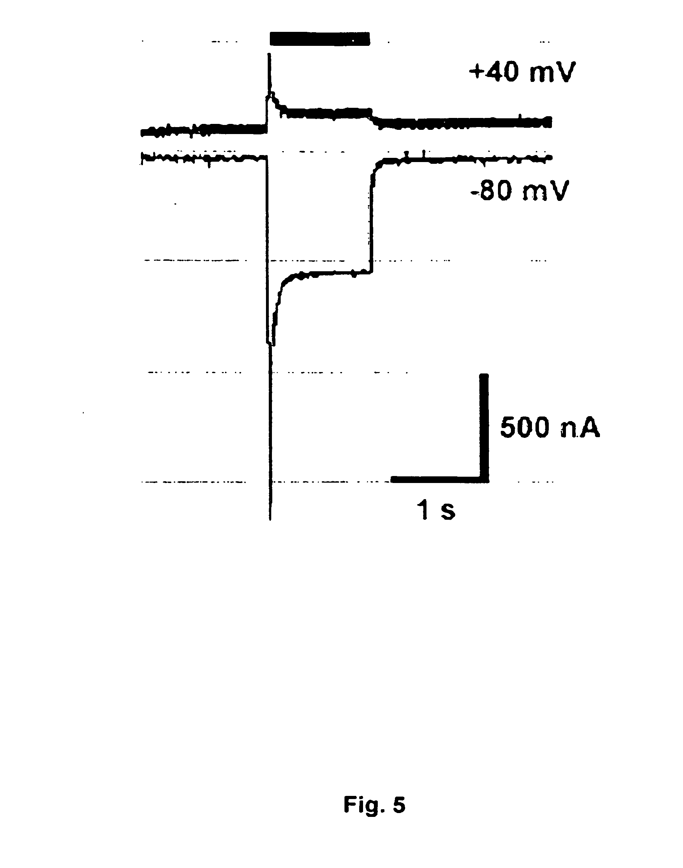

Ähnlich wie bei dem CHOP-1 Protein, konnte auch hier gezeigt werden, dass eine verkürzte Form von CHOP-2, welche die Aminosäuren 1-315 umfasst, ebenfalls einen funktionsfähigen lichtgeschalteten Ionenkanal bildet (Fig. 5-7).

CHOP-2 und oder CHOP-2-315 können zur Depolarisation von Zellen (siehe Fig. 7) oder zur Ca²⁺-Aufnahme (Fig. 6) oder zu beidem benutzt werden.

Im Rahmen der vorliegenden Erfindung ist der Fachmann in der Lage, Derivate der ursprünglichen Aminosäuresequenz in Kenntnis der Nukleotid- und Aminosäuresäuresequenz sowie der berechneten 3D-Struktur des natürlichen CHOP-2-Proteins, durch Austausch und/oder Insertion und/oder Deletion von einer oder mehreren Aminosäure(n) herzustellen, welche die lonentransporteigenschaften des ursprünglichen Proteins ganz oder teilweise behalten haben oder sich bezüglich lonenspezifität, Inaktivierung, Gleichrichtung etc. signifikant unterscheiden. Derivate sind dabei wie oben angegeben definiert.

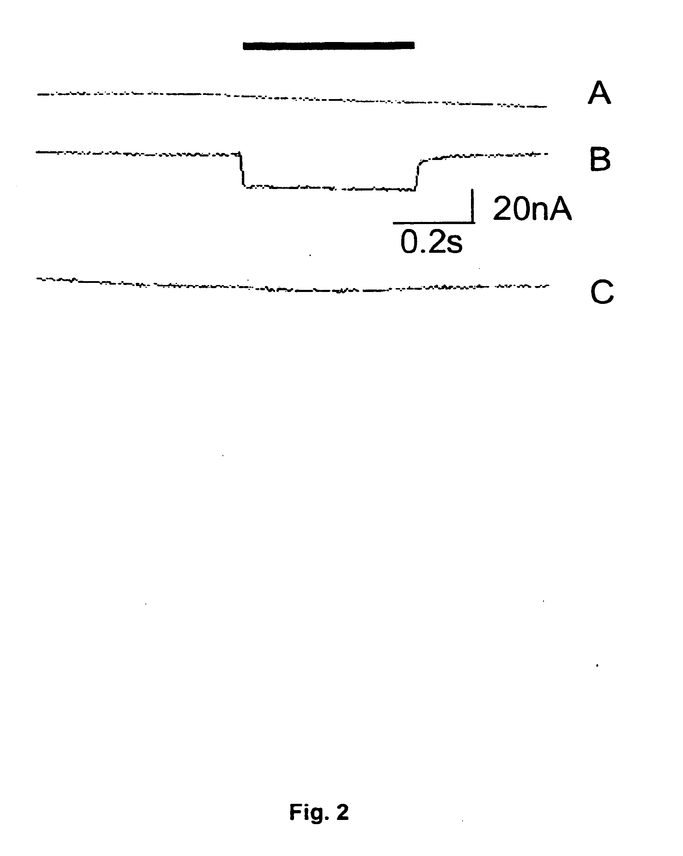

Aktive lonentransportsysteme können von passiven Systemen elektrophysiologisch eindeutig und zweifelsfrei unterschieden werden, wie nachfolgend ausgeführt. Passive lichtgesteuerte Ionenkanäle, die Gegenstand der Erfindung sind, erhöhen im Licht die Leitfähigkeit von Membranen, in die sie eingebaut sind, gegenüber dem Dunkelzustand. Die Ionenströme, die bei Belichtung auftreten (Photoströme), werden von der Ionenspezies getragen, die von dem lichtgesteuerten Ionenkanal geleitet wird. Bei gleicher Konzentration der geleiteten Ionen (bei CHOP-1 Protonen; bei CHOP-2 z.B. Protonen) auf beiden Seiten der Membran (symmetrische Bedingungen) wird die Richtung des Photostroms allein durch das angelegte Membranpotential bestimmt. Im Falle eines positiven Ladungsträgers erfolgt bei negativem Potential (in der Zelle negativ) ein Einwärtsstrom unter Bewegung der positiv geladenen Ionen in die Zelle, bei positivem Potential (innen positiv) ein Auswärtsstrom unter Bewegung der positiv geladenen Ionen aus der Zelle heraus. Die Strom-Spannungskurven eines erfindungsgemäßen lichtgesteuerten Ionenkanals gehen bei symmetrischer Verteilung des transportierten Ions immer durch den Nullpunkt (Umkehrpotential E_{H}=0). Die Strom-Spannungskurven der erfindungsgemäßen lichtgesteuerten Ionenkanäle sind jedoch nicht notwendigerweise linear. In CHOP-1 zum Beispiel wird der Strom in Einwärtsrichtung besser geleitet als in Auswärtsrichtung (Einwärtsgleichrichter, siehe Fig. 2 und 3).

Im Gegensatz dazu geht bei allen aktiven Transportsystemen (wie z. B. Bakteriorhodopsin) die Strom-Spannungskurve bei symmetrischer Ionenverteilung nie durch Null. Die Einstellung einer Ungleichverteilung der geleiteten Ionen zwischen innen und außen hat eine Verschiebung der Strom-Spannungskurven entlang der Spannungsskala entsprechend der Nernst'schen Gleichung zur Folge, d.h., um 58 mV bei monovalenten Ionen, um 29 mV bei divalenten Ionen.

Durch Untersuchung der Strom-Spannungskurven kann daher für jeden lichtgesteuerten Ionenkanal festgestellt werden, ob es sich um ein passives oder aktives System handelt.

Die vorliegende Erfindung betrifft daher Derivate und Fragmente der CHOP-2 Proteine, welche die lonentransporteigenschaften des ursprünglichen Proteins ganz oder teilweise behalten haben oder sogar in gesteigertem Maße aufweisen, sowie strukturell und funktionell ähnliche Opsine aus anderen Organismen, natürlicher Herkunft oder durch Anwendung rekombinanter Techniken modifiziert, wenn sie die genannten biophysikalischen Eigenschaften aufweisen und wie CHOP-2 zu den genannten Zwecken eingesetzt werden können, wie in den Ansprüchen definiert.

Umfangreiche Versuchsreihen zur Herstellung und Untersuchung solcher Derivate führten zu dem überraschenden Ergebnis, dass die Aminosäureposition 134 im CHOP-2-Protein für die Funktion als lichtgeschalteter Ionenkanal von besonderer Bedeutung ist. So kann beispielsweise durch Austausch von Histidin an Position 134 gegen Arginin eine besonders aktive Mutante des vollständigen CHOP-2-Proteins bzw. des verkürzten CHOP-2-315-Proteins erzeugt werden. Dieser Austausch kann nach Standardverfahren z. B. durch stellengerichtete Oligonukleotid-Mutagenese durchgeführt werden. Weitere Daten legen nahe, dass Glu123 an einer lichtinduzierten Deprotonierung beteiligt ist, die zu einer Desensibilisierung des Ionenkanals führt. So erhöhte die Mutation von Glu123 zu Asp (E123D) das Verhältnis des transienten Photostroms zu dem Gleichgewichts-Photostrom deutlich, während die Mutation von Glu123 zu Gln (E123Q) den Peak des transienten Photostroms fast verschwinden lässt, aber auch zu einer starken Verringerung des stationären Photostroms führt.

Das erfindungsgemäße passive lonentransportsystem enthält ein lichtsensitives Polyen. Dabei kann es sich beispielsweise um p-Hydroxyzimtsäure, Retinal oder ein Retinalderivat handeln. Bevorzugte Retinalderivate sind aus der folgenden Gruppe ausgewählt: 3,4-Dehydroretinal, 13-Ethylretinal, 9-dm-Retinal, 3-Hydroxyretinal, 4-Hydroxyretinal, Naphthylretinal; 3,7,11-Trimethyl-dodeca-2,4,6,8,10-Pentaenal; 3,7-Dimethyl-deca-2,4,6,8-tetraenal; 3,7-Dimethyl-octa-2,4,6-trienal; sowie 6-7-, oder 10-11-rotations-blockierte Retinale mit 4-, 5-, 6- oder 7-Ringverbrückungen. Besonders bevorzugt ist jedoch ein 10-12-Fünfring-verbrücktes Retinal (Takahashi et al. FEBS Lett. 314, 275-279).

Nach Lichtabsorption und Isomerisierung der Polyene kommt es in den erfindungsgemäßen lichtgesteuerten Ionenkanälen zu einer strukturellen Änderung im Protein (Opsin) und damit zur Öffnung des Ionen leitenden Kanals, der die intrazelluläre Seite der Membran mit der extrazellulären verbindet. Dieses Ereignis unterscheidet sich grundsätzlich von der Situation in den bekannten Ionenpumpen, bei denen der extrazelluläre protonenleitende Halbkanal (EC) mit dem intrazellulären Halbkanal (IC) nie leitend verbunden ist. Im Bakteriorhodopsin ist die Schiffsche Base des Retinal im frühen M-Intermediat des Reaktionszyklus (Photozyklus) mit der extrazellulären Seite leitend verbunden, im späten M-Zustand dagegen ist sie mit der intrazellulären Seite verbunden.

Die erfindungsgemäßen lichtgesteuerten Ionenkanäle können in eine Membran, beispielsweise die Plasmamembran einer Zelle, eingebaut und dazu verwendet werden, das Membranpotential durch Belichtung schnell und definiert zu verändern, was zur Aufklärung der Mechanismen von spannungsabhängigen Ionenkanälen oder Ionentransportern sehr hilfreich ist. Ferner ergibt sich so die Möglichkeit, mit einem lichtgesteuerten gezielten Ionentransport die intrazellulären Niveaus dieser Ionen schnell und definiert zu verändern.

Im Falle des CHOP-1-Proteins bedeutet dies eine gezielte nicht invasive Veränderung des intrazellulären pH-Werts, was zur Aufklärung der Mechanismen der intrazellulären pH-Regulation bzw. des Einflusses von transienten pH-Änderungen auf zelleigene Proteine von Nutzen ist.

Durch die Messung des Umkehrpotentials der lichtinduzierten CHOP-1-vermittelten Protonenleitfähigkeit kann der intrazelluläre pH-Wert direkt unterhalb der Membran, bzw. ein pH-Gradient über die Membran bei Kenntnis des extrazellulären pHs schnell und genau gemessen werden. Hierzu wird mit der in Fig. 2-4 angewandten "Voltage Clamp" Messung das Umkehrpotential bestimmt und damit die Zellen geeicht. Nachfolgend kann die Modulation des Membranpotentials und/oder des intrazellulären pH-Werts mit Licht über den erfindungsgemäßen lichtgesteuerten Ionenkanal nichtinvasiv erfolgen. Die entsprechenden Messungen erfolgen schnell und definiert und eignen sich so in idealer Weise für moderne HTS (High-Throughput-Screening)-Geräte, mit denen sich beispielsweise pH-regulierte spannungsabhängige Membranproteine wie Ionenkanäle in Screeninganwendungen mit hohem Durchsatz testen lassen. Spannungs- oder pH-Sprünge lassen sich in Zelllinien, die ChR1 oder verwandte Rhodopsine enthalten, mit Licht induzieren.

Eine besonders interessante Anwendung ist die optoelektrische Kopplung durch lichtgesteuerte Modulation des Membranpotentials. Diese Anwendung beruht auf einer Kombination des lichtgesteuerten passiven Ionenkanals mit einem lichtgesteuerten aktiven lonentransportsystem, z.B. einer Ionenpumpe, wobei zur Lichtsteuerung des passiven Ionenkanals vorzugsweise eine andere Wellenlänge als zur Lichtsteuerung des aktiven lonentransportsystems eingesetzt wird. In einer bevorzugten Ausführungsform wird Licht einer Wellenlänge von 650 nm zur Aktivierung der Protonenpumpe Bakteriorhodopsin und zum Aufbau eines Membranpotentials eingesetzt und anschließend Licht einer Wellenlänge von 440 nm, das auf Bakteriorhodopsin inhibitorisch wirkt, zur Aktivierung des CHOP-1-Protonentransportsystems und zur schnellen Dissipation des Potentials eingesetzt.

Lichtregulierte Ionenkanäle können auch bei der Signalübertragung von neuronalen Netzwerken auf Mikroelektrodennetze eingesetzt werden. Hierbei wird versucht, elektrische Impulse von Neuronen auf Mikrocomputer zu übertragen ("Interfacing von Nervenzellen und Halbleitern": Fromherz (2001), Physikalische Blätter 57, 43-48). Bislang mussten die Neuronen entweder über Neurotransmitter oder direkt mit Mikropipetten stimuliert werden. Neuronen, die ChR2 oder verwandte lichtgesteuerte Ionenkanäle exprimieren, könnten über Licht gesteuert werden.

Eine weitere Anwendung ist die Therapie von blinden Tieren oder letztendlich Menschen. Es gibt einige Krankheiten, bei denen die natürlichen Sehzellen nicht mehr funktionieren, aber alle Nervenverbindungen weiterhin arbeiten können. Heute wird in verschiedenen Forschungszentren versucht, dünne Filme mit künstlichen keramischen Photozellen auf der Netzhaut zu implantieren (E. Zrenner (2002), Science 295, 1022-1025.) Diese Photozellen sollen dazu dienen, die sekundären noch intakten Zellen der Retina zu depolarisieren und damit einen Nervenimpuls auszulösen (*bionische Augen*). Die gezielte Expression von lichtgesteuerten Rhodopsinen wie ChR1 oder ChR2 in diesen Ganglionzellen, Amacrinzellen oder bipolaren Zellen würde eine sehr viel elegantere Lösung darstellen und eine höhere räumliche visuelle Auflösung ermöglichen.

Der Einbau des erfindungsgemäßen Rhodopsin-lonentransportsystems in die Membran von Zellen, welche das entsprechende Opsin-Protein in der Natur nicht exprimieren, kann z.B. in einfacher Weise dadurch erfolgen, dass die für dieses Opsin kodierende DNA unter Anwendung bekannter Verfahren der rekombinanten DNA-Technologie zuerst in einen geeigneten Expressionsvektor, z. B. ein Plasmid, ein Cosmid oder ein Virus, eingebaut wird, damit anschließend die Zielzellen transformiert werden und das Protein in diesem Wirt exprimiert wird. Anschließend werden die Zellen in geeigneter Weise z.B. mit Retinal behandelt, um die Verbindung einer Schiffschen Base zwischen Protein und Retinal zu ermöglichen.

In einer bevorzugten Ausführungsform geschieht dieses in verschiedenen Hefen wie *Saccharomyces cerevisiae, Schizosaccharomyces pombe, Pichia pastoris* wie bereits für aktive Rhodopsine wie Bakteriorhodopsin und/oder Rinderrhodopsin erfolgreich durchgeführt (Hildebrandt V. et al. (1989)), Genetic transfer of the pigment bacteriorhodopsin into the eukaryote Schizosaccharomyces pombe, FEBS Lett. 243(2):137-40; Najmoutin G. et al., Heterologous expression of bovine opsins in Pichia pastoris, Meth. Enzymol. (2000) 315, 3-11).

Die Expression kann auch in bestimmten Säugerzellsystemen erfolgen. Dieses geschieht entweder mit episomalen Vektoren als transiente Expression, vozugsweise in COS-Zellen (generiert durch Infektion von "African green monkey kidney CV1" Zellen) (Oprian et al. (1987) Proc Natl. Acad. Sci USA 84, 8874 ff.) oder HEK-Zellen ("*human embyonic kidney* cells", z.B. HEK293-Zellen, Reeves et al. (1996) Proc. Natl. Acad. Sci USA 84, 11487 ff.) oder BHK-Zellen ("baby hamster kidney cells"), oder in Form einer stabilen Expression (durch Integration ins Genom) in CHO-Zellen ("chinese hamster ovary cells"), Myelom-Zellen oder MDCK-Zellen ("Madine-Darby canine kidney cells") (Übersicht in: Makrides SC (1999) Prot. Expr. Purif. 17, 183-202) oder in durch Baculoviren infizierte Sf9-Insektenzellen (Jansen et al. (1988) Mol Biol. Rep. 13, 65 ff.).

Zur Gewährleistung oder Optimierung der Expression kann die kodierende DNA auch in geeigneter Weise modifiziert werden, beispielsweise durch Verknüpfung mit geeigneten regulatorischen Sequenzen und/oder durch Anpassung der kodierenden DNA-Sequenz an die bevorzugte Codonverwendung des gewählten Expressionssystems.

### Figurenbeschreibung:

Fig. 1
Fig. 1A: Aminosäuresequenz des Channelopsin1 (Chop1) SEQ ID NO: AF385748
Fig. 1B: Aminosäuresequenz des Channelopsin2 (Chop2) SEQ ID NO: AF461397
Fig. 1C: Aminosäuresequenz des Bacterioopsins (Bop) aus *Halobacterium salinarium* (BR). Mit kleinen Buchstaben wird die *Leadersequenz* angezeigt, die *in vivo* abgespalten wird und aus historischen Gründen bei der Nummerierung der Aminosäuren nicht mitgezählt wird. In fetten Buchstaben sind die für die Protonenleitung essentiellen Aminosäuren gezeigt.
Fig. 1D: Vergleich der Aminosäuresequenzen von CHOP-1 (SEQ ID NO: AF385748) und CHOP-2 (SEQ ID NO: AF461397) aus *Chlamydomonas reinhardtii* mit der von Bakteriorhodopsin aus *Halobacterium salinarum*. Aminosäuren, von denen für BR (Lücke et al. (1999) Science 286, 255-260 und darin erwähnte Literatur) bekannt ist, dass sie direkt mit dem Retinal wechselwirken, sind durch Sterne angezeigt. Aminosäurepositionen, die in mindestens zwei Sequenzen gleich sind, sind hellgrau unterlegt. Aminosäuren, die zu dem H⁺-leitenden Netzwerk in BR beitragen und die diesen entsprechenden Aminosäuren in den anderen Opsinen sind weiß auf schwarzem Grund. Für das His 173 von CHOP-1 wurde im Rahmen der Erfindung gezeigt, dass es an der Protonenleitung beteiligt ist. # indiziert die Position des Retinal-bindenden Lysins. Die unterstrichenen Aminosäuren zeigen die 7 Transmembran-Helices des Core-Proteins an.
Fig. 1E: Aminosäuresequenz der CHOP-2-Core-Protein-Mutante CHOP2-315/-H134R), bei der Histidin an Position 134 durch Arginin ersetzt ist.
Fig. 2 Photoströme, die während der Belichtung von Oozyten mit grünem oder rotem Licht (500 ± 25 nm bzw. 7.00 ± 25 nm, 10²² Photonen, m⁻² s⁻¹) registriert wurden. Haltepotential (Vₕ) = -100 mV, Lichtpuls durch den Balken angezeigt. Badlösung = 96 mM NaCl, 5 mM KCI, 2 mM CaCl₂, 1 mM MgCl₂, 5 mM MOPS, pH 7,5. A) Eine Oozyte, der keine CHOP-1-RNA injiziert wurde, bestrahlt mit grünem Licht B) eine CHOP-1-Oozyte, bestrahlt mit grünem Licht; C) dieselbe Oozyte wie in B), bestrahlt mit rotem Licht.
Fig. 3 Abhängigkeit des lichtinduzierten Einwärtsstroms von den lonenbedingungen und der Spannung.
a: Ergebnisse mit einer (von fünf) charakteristischen CHOP-1-Oozyte, in der Reihenfolge der Messung (etwa 150 s Intervall), dargestellt, Vₕ = -100 mV, grünes Licht wie in Fig. 2. Die Lösungen sind mit 5 mM MOPS (pH 7,5) oder MES (pH 6) oder Citrat (pH 5 und 4) gepuffert. Konzentration in mM:

| | |
|---|---|
| 1. 100 NaCl, 2 CaCl₂, pH 7,5 | 2: 100 NaCl, 2 CaCl₂, pH 6,0 (Referenz) |
| 3: 100 Na-Aspartat, 2 CaCl₂, pH 6,0 | 4: 100 NMG-Cl, 2 CaCl₂, pH 6,0 |
| 5: wie 2 | 6: 100 NaCl, 2 EGTA, 2 MgCl₂, pH 6,0 |
| 7: 200 Sorbit, 5 EGTA, pH 5,0 | 8: 200 Sorbit, 5 EGTA, pH 4,0 |

b: Strom-Spannungs-Verhältnisse der Photoströme von Fig. 3a, Konzentrationen siehe oben.
Fig. 4 Photoströme, die unter Variation des äußeren und inneren pH-Werts aufgezeichnet wurden, und deren Abhängigkeit von der Wellenlänge
a: Photostrom bei Vₕ = +40 mV. Ergebnisse von einer (von fünf) charakteristischen CHOP-1-Oozyten in einer Badlösung von NaCl (100 mM, Spur A: rotes Licht; Spur B: grünes Licht; 10²² Photonen m⁻² s⁻¹) oder Natriumbutyrat (40 mM, +60 mM NaCl, Spur C: grünes Licht) von pH 7,4
b: Strom-Spannungs-Verhältnisse von Photoströmen in verschiedenen Badlösungen, die immer 100 µM undissoziierte Buttersäure enthielten:
   ■, 60 mM NaCl + 40 mM Na-Butyrat, pH 7,4; ▲, 84 mM NaCl + 16 mM Na-Butyrat, pH 7,0; ▼, 93,6 mM NaCl + 6,4 mM Na-Butyrat, pH 6,6; ◆, 97,4 mM NaCl + 2,6 mM Na-Butyrat, pH 6,2;
**c**: pH-Abhängigkeit von Umkehrpotentialen aus b. Die punktierten Linien zeigen die theoretische Beziehung für einen konstanten pHᵢ von 6,6 bzw. 6,8 und -58 mV/pH. Die gestrichelte Linie zeigt die erwartete Beziehung für eine Steigung von -48 mV/pH-Differenz und pHᵢ = 6,8 bei pHₒ = 7,4. Die Steigung von -48 mV/pH entspricht einem langsam abnehmenden pHᵢ (um 0,17 Einheiten pro pHₒ-Abnahme von einer Einheit). □, pH-Abhängigkeit von Umkehrpotentialen aus Versuchen mit 5,4 mM undissoziierter Essigsäure (n =3). Die punktierte Linie zeigt die theoretische Beziehung für einen konstanten pHᵢ von 5,5 und -58 mV/pH.
d: Wellenlängenabhängigkeit des lichtinduzierten Einstromes bei pHₒ 5,5 und - 40 mV. Die Photoströme wurden für einen gleichen Photonenstrom normiert.
   - Fig. 5: Photoströme, die bei der Belichtung (Wellenlänge 450 ± 25 nm) von CHOP2-315 exprimierenden Oozyten in Ringerscher Lösung (110 mM NaCl, 5 mM KCI, 2 mM CaCl₂, 1mM MgCl₂, 5 mM MOPS, pH 7,6) bei +40 mV und -80 mV registriert wurden.

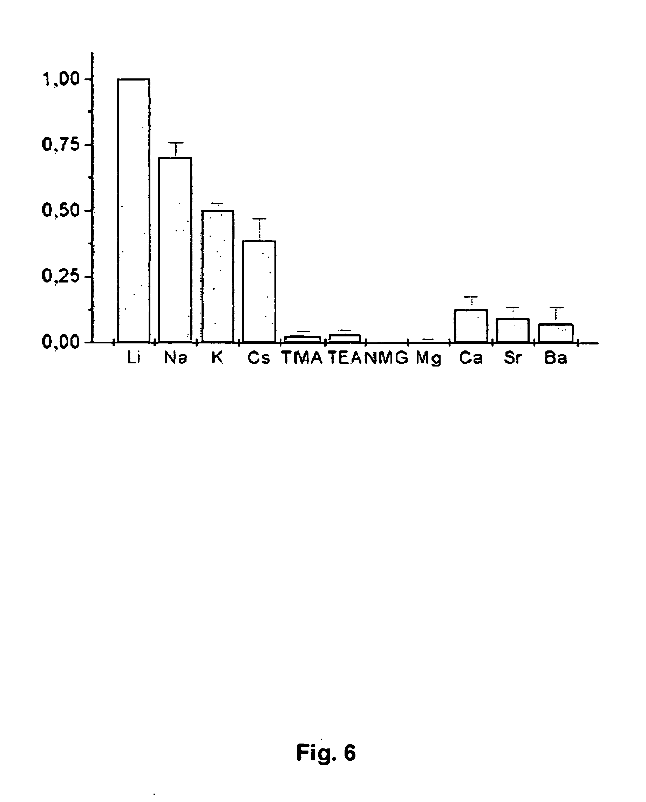

   - Fig. 6: Größe des lichtinduzierten Gleichgewichts-Einstroms bei -100 mV, pH 9, für 115 mM Li, Na, Cs, TMA, TEA oder NMG und für 80 mM Mg²⁺, Ca²⁺, Sr²⁺ oder Ba²⁺ (für beide Varianten CHOP2 und CHOP2-315 identisch)

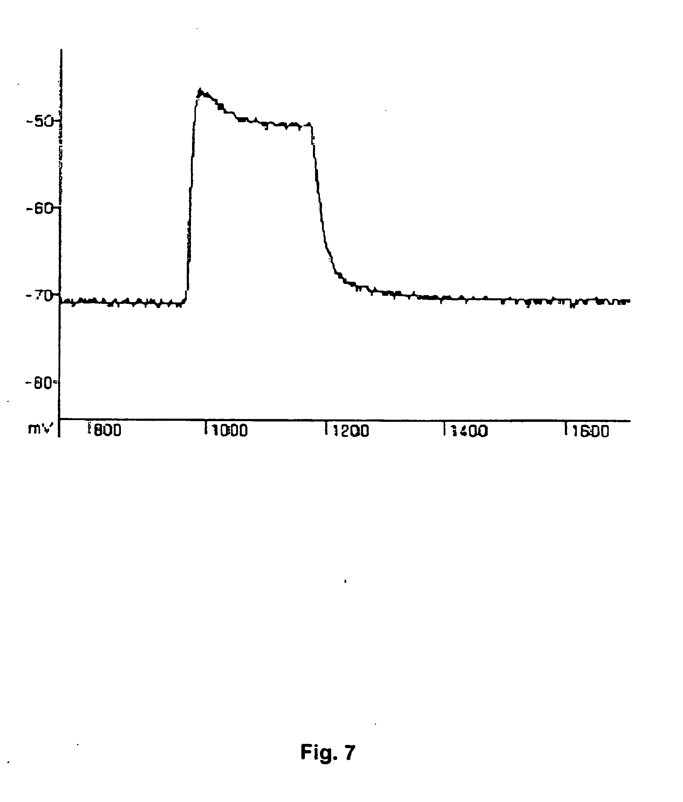

   - Fig. 7: Depolarisation einer Oozyten-Plasmamembran, die CHOP2-315 exprimiert, durch einen Lichtblitz von 200 ms

Die folgenden Beispiele erläutern die vorliegende Erfindung, ohne diese jedoch darauf zu beschränken.

### BEISPIEL I (nicht beansprucht)

### Amplifizierung von CHOP-1-DNAs und Expression von funktionsfähigen CHOP-1-Photorezeptoren in Xenopus laevis

Eine CHOP-1-DNA vollständiger Länge, kodierend für die Aminosäuren 1-712, und zwei kürzere CHOP-1-DNAs, kodierend für die Aminosäuren 1-346 bzw. 1-517 von CHOP-1, wurden von einer cDNA-Matrize vollständiger Länge mittels PCR unter Verwendung einer Polymerase mit Korrekturfunktion (pfu, Promega) und zwei Startoligonukleotiden (Primern), die BamHI- und HindlII-Restriktionsstellen enthielten, amplifiziert. Die Produkte wurden in den Vektor pGEMHE (Liman et al., Neuron 9, 861-71 (1992)) inseriert und in *E. coli* kloniert. Nach Überprüfung der Sequenzen wurden die CHOP-1-DNAs *in vitro* transkribiert (Ambion) und 20 bis 30 ng cRNA in Oozyten von *Xenopus laevis* injiziert, wie bereits für Bakteriorhodopsin beschrieben (Nagel et al., FEBS Letters, 377, 263-266 (1995). Die Oozyten, die das CHOP-1-Protein exprimierten, wurden mit einem *all*-*trans*-Retinal (1 µM) in Ringerscher Lösung zwei bis fünf Tage lang inkubiert.

### BEISPIEL II (nicht beansprucht)

### Charakterisierung des CHOP-1-Photorezeptors

Zur Untersuchung der angenommenen lonentransportfunktion wurden die CHOP-1-exprimierenden Oozyten unter Anwendung einer Zweielektroden-Spannungsklemmen-Technik, die bereits für Bakteriorhodopsin verwendet worden war (Nagel et al. FEBS Letters, 377, 263-266 (1995); Nagel et al., Biophysical Journal 74, 403-412 (1998)) verschiedenen Experimenten unterworfen. Grünes Licht, nicht jedoch rotes Licht, induzierte einwärts gerichtete Ströme bei Oozyten, die eine der CHOP-1-RNAs exprimierten (Fig. 2). Das Auftreten von Photoströmen auch bei den kürzeren CHOP-1-RNAs demonstrierte, dass ein großer Teil des carboxyterminalen Bereichs von CHOP-1 für diese Funktion nicht erforderlich ist. Bei pH 6 und einer Transmembranspannung zwischen -100 und + 40 mV waren die Photoströme immer einwärts gerichtet (Fig. 3b). Der Ersatz von Chlorid durch Aspartat in der Lösung hatte keine nachweisbare Wirkung auf die Amplitude des Photostroms (Fig. 3a) oder dessen Strom-Spannungsverhältnis (Fig. 3b), ein Ergebnis, das Cl⁻ als transportiertes Ion ausschloss. Der Ersatz von Natrium durch N-Methyl-D-glucamin (oder von NaCl durch Sorbit, Daten nicht gezeigt) führte zu einem ähnlichen einwärts gerichteten Strom (Fig. 3a) ohne Veränderung des Strom-Spannungsverhältnisses (Fig. 3b), was anzeigt, dass Na⁺ von CHOP-1 nicht transportiert wird. Gleichermaßen ergab der Ersatz von Ca²⁺ durch Mg²⁺ keine Veränderung der Photoströme, ein Ergebnis, welches zeigte, dass Ca²⁺ ebenfalls nicht das transportierte Ion war (Fig. 3a,b).

Dagegen führte eine Erhöhung der Protonenkonzentration in der Badlösung, [H⁺]ₒ, auf pH-Werte von 5 bzw. 4 bei Potentialen zwischen -100 und +40 mV zu deutlichen Anstiegen der einwärts gerichteten Photoströme (Fig. 3a,b).

Die bisher erhaltenen Ergebnisse verweisen somit auf H⁺-Ionen als Ladungsträger der lichtinduzierten Ströme. Aufgrund der eingangs erwähnten Sequenzhomologien zwischen dem CHOP-1-Protein und der Protonenpumpe Bakteriorhodopsin lag zunächst die Annahme nahe, dass auch das CHOP-1-Protein Bestandteil eines aktiven lonentransportsystems, nämlich einer Protonenpumpe wie Bakteriorhodopsin, wäre.

Während Bakteriorhodopsin jedoch bei allen getesteten Membranpotentialen von - 60 mV bis + 40 mV (siehe z.B. Nagel et al., Biophysical Journal 74, 403-412 (1998)) auch gegen einen bestehenden pH-Gradienten Protonen immer nach außen transportiert, war die Transportrichtung des CHOP-1-Systems abhängig von dem über die Membran vorliegenden pH-Gradienten und dem Membranpotential (Fig. 4a,b,c). Die gemessenen Umkehrpotentiale bei verschiedenen Ausgangs-pH-Gradienten (Fig. 4b,c) belegen eindeutig, dass die lichtinduzierten Ströme rein passiver Natur sind. Aus den beobachteten hohen Photoströmen ist zu schließen, dass der Protonentransport nicht nur durch eine erleichterte Diffusion der Protonen durch die Membran vonstatten geht, sondern dass das CHOP-Protein ein Protonenkanal ist.

Die Abhängigkeit des lichtinduzierten einwärts gerichteten Photostroms bei pH 5,5 und -40 mV von der Wellenlänge des Lichts ist in Fig. 4d dargestellt. Das Maximum in der Nähe von 500 nm entspricht den Aktionsspektren für Photorezeptorströme, für Phototaxie- und Photoschock-Reaktionen intakter *C. reinhardtii*-Zellen.

### BEISPIEL III

### Amplifizierung von CHOP-2-DNAs und Expression von funktionsfähigen CHOP-2-Photorezeptoren in Xenopus laevis

Eine CHOP-2-DNA vollständiger Länge, kodierend für die Aminosäuren 1-737, und eine C-terminal verkürzte CHOP-2-DNA, kodierend für die Aminosäuren 1-315 von CHOP-2, wurden von einer cDNA-Matrize vollständiger Länge mittels PCR unter Verwendung einer Polymerase mit Korrekturfunktion (pfu, Promega) und Primern, die BamHI- und Hindlll-Restriktionsstellen enthielten, amplifiziert. Die Produkte wurden in den Vektor pGEMHE (Liman et al., Neuron 9, 861-71 (1992)) inseriert und in *E. coli* kloniert. Nach Überprüfung der Sequenzen wurden die CHOP-2-DNAs *in vitro* transkribiert (Ambion) und 20 bis 30 ng cRNA in Oozyten von *Xenopus laevis* injiziert, wie bereits für Bakteriorhodopsin beschrieben (Nagel et al., FEBS Letters, 377, 263-266 (1995). Die Oozyten, die das CHOP-2-Protein exprimierten, wurden mit einem *alltrans*-Retinal (1 µM) in Ringerscher Lösung zwei bis fünf Tage lang inkubiert.

### BEISPIEL IV

### Charakterisierung der CHOP-2-Photorezeptoren

Zur Untersuchung der lonentransportfunktion wurden die CHOP-2-exprimierenden Oozyten, die zur Bildung des ChR2 (Channelrhodopsin2)-lonenkanals in Ringer scher Lösung mit Retinal inkubiert worden waren, unter Anwendung ähnlicher Zweielektroden-Spannungsklemmen-Techniken, wie sie bereits für CHOP-1 bzw. ChR1 verwendet worden waren, verschiedenen Experimenten (Fig. 5-7) unterworfen.

Fig. 5 zeigt, dass der lichtinduzierte Photostrom bei Bestrahlung mit Dauerlicht (Wellenlänge 450 ± 25 nm; entspricht etwa dem Maximum des Aktionsspektrums) auf ein Gleichgewichtsniveau abfällt. Dies bedeutet, dass der ChR2-lonenkanal durch Dauerlicht dieser Wellenlänge desensibilisiert wird, möglicherweise durch eine Inaktivierung eines Teils der ChR2-Moleküle.

Fig. 6 demonstriert, dass der mit CHOP-2 gebildete Ionenkanal im Gegensatz zu dem mit CHOP-1 gebildeten Ionenkanal einen nicht-selektiven Kationenkanal darstellt, der neben Protonen auch verschiedene ein- und zweiwertige Kationen passieren lässt. Die Leitfähigkeit für die untersuchten einwertigen Kationen nimmt dabei in der Reihenfolge Li⁺ > Na⁺ > K⁺ > Cs⁺ >> TMA⁺ (Tetramethylammonium) = TEA⁺ (Tetraethylammonium), d.h., mit zunehmendem Ionenradius, ab. Bei zweiwertigen Kationen nimmt die Leitfähigkeit ebenfalls mit zunehmenden Ionenradius ab. Hier stellt jedoch Mg²⁺ die Ausnahme dar. ChR2 ist für Mg²⁺ nicht durchlässig, wahrscheinlich aufgrund der hohen Hydratationsenergie dieses Ions (Diebier et al., Pure Appl. Chem. 20, S. 93, 1969). Die jeweiligen Photoströme für den mit CHOP-2 voller Länge gebildeten Ionenkanal unterschieden sich dabei nicht von den entsprechenden Photoströmen für den Ionenkanal, der das CHOP-2-Fragment mit den aminoterminalen Aminosäuren 1-315 umfasst, was die Schlussfolgerung erlaubt, dass die Kationenleitfähigkeit durch den aminoterminalen Anteil des ChOP-2-Proteins, welcher die sieben mutmaßlichen Transmembrandomänen enthält, bestimmt wird.

Diese und weitere Aspekte der Erfindung werden anhand der folgenden Erläuterungen weiter beschrieben, wobei die Nennung der Eigenschaften eines biologischen Photorezeptors auch dessen entsprechend ausgeprägte Verwendungen mit beinhalten soll:

Geschaffen werden ein biologischer Photorezeptor, insbesondere als direkt lichtgesteuerter Ionenkanal zur Veränderung der lonenleitfähigkeit einer Membran mit Hilfe von Licht, und dessen entsprechende Verwendungen, wobei der Photorezeptor ein Apoprotein und ein kovalent an das Apoprotein gebundenes lichtsensitives Polyen, welches mit dem Apoprotein wechselwirkt und als lichtempfindliches Tor fungiert, aufweist, wobei das Apoprotein ein Ionenkanal bildendes Opsin oder ein Derivat oder Fragment eines Ionenkanal bildenden Opsins ist.

Das Apoprotein kann ein Transmembranprotein mit fünf oder mehr Transmembranhelices sein.

Das Apoprotein ist ein Derivat oder ein Fragment eines Channelopsin2 (CHOP-2), wie in den Ansprüchen definiert.

Das lonentransportsystem das gebildet wird kann ein Protonentransportsystem sein.

Das Opsin-Derivat oder Opsin-Fragment kann das Ergebnis eines Austausches, einer Insertion und/oder Deletion von einer Aminosäure oder von mehreren Aminosäuren in der natürlichen Aminosäuresequenz des Opsin-Proteins sein, wie in den Ansprüchen definiert.

Die dem Bakteriorhodopsin-Asp96 entsprechende Aminosäure kann eine andere Aminosäure als Asp sein. In dem Apoprotein können mindestens acht der weiteren sechzehn Aminosäuren, die im Bakteriorhodopsin am Protonen¬transportnetzwerk beteiligt sind, identisch erhalten oder durch konservativen Austausch verändert sein.

Mindestens die Aminosäuren, die im Bakteriorhodopsin den Aminosäuren T46, Y57, R82, T89, T107, W182, D212, K216 entsprechen, können an der entsprechenden Position identisch erhalten sein.

Das Apoprotein kann die Konsensussequenz L(I)DxxxKxxW(F,Y) enthalten.

Das Apoprotein kann aus niederen Pflanzen stammen.

Die niederen Pflanzen können Algen sein.

Das Apoprotein kann ein Opsin-Protein aus Chlamydomonas reinhardtii sein.

Das Apoprotein umfasst mindestens die Aminosäuren 24 bis 268 des Channelopsin2 (CHOP-2) gemäß SEQ ID NO:AF461397 (National Center for Biotechnology Information, NCBI) oder gemäß der in Fig. 1B gezeigten Aminosäuresequenz.

Die Aminosäure Histidin an Position 134 des Channelopsin2 (CHOP2-2) kann durch eine andere Aminosäure ersetzt sein.

Die Aminosäure Histidin an Position 134 des Channelopsin2 (CHOP2-2) kann durch Arginin ersetzt sein.

Das lichtsensitive Polyen kann ein Retinal oder Retinalderivat sein.

Das Retinalderivat kann aus der folgenden Gruppe ausgewählt sein: 3,4-Dehydroretinal, 13-Ethylretinal, 9-dm-Retinal, 3-Hydroxyretinal, 4-Hydroxyretinal, Naphthylretinal; 3,7,11-Trimethyl-dodeca-2,4,6,8,10-pentaenal; 3,7-Dimethyl-deca-2,4,6,8-tetraenal; 3,7-Dimethyl-octa-2,4,6-trienal; sowie 6-7- oder 8-9- oder 10-11-Rotations-blockierte Retinale.

Der biologische Photorezeptor kann zur lichtgesteuerten Veränderung der Protonenleitfähigkeit der Membran ausgebildet sein.

Der biologische Photorezeptor kann zur lichtgesteuerten Veränderung des Membranpotentials einer Zelle ausgebildet sein.

Bei der Membran kann es sich um die Zellmembran einer Hefe, z.B. Saccharomyces cerevisiae, Schizosaccharomyces pombe, Pichia pastoris, handeln.

Bei der Membran kann es sich um die Zellmembran einer Säugerzelle oder Insektenzelle, z.B. COS, BHK, HEK293, CHO, Myelom-Zelle, MDCK, Baculovirus-infizierte sf9-Zelle, handeln.

Der biologische Photorezeptor kann zur lichtgesteuerten Erhöhung oder Erniedrigung der intrazellulären Konzentration von Ionen ausgebildet sein.

Der biologische Photorezeptor kann zur lichtgesteuerten Erhöhung oder Erniedrigung der intrazellulären Protonenkonzentration ausgebildet sein.

Der biologische Photorezeptor kann zur Messung der intrazellulären Protonenkonzentration direkt an der Plasmamembran oder eines Protonenkonzentrationsgradienten über der Plasmamembran mit Hilfe von Strom-Spannungskurven ausgebildet sein, wobei der Protonenkonzentrationsgradient aus der Differenz der Strom-Spannungskurven mit und ohne Belichtung aus dem Umkehrpotential direkt ermittelt werden kann

Der biologische Photorezeptor kann zum Hochdurchsatzscreening von biologischen Molekülen ausgebildet sein

Der biologische Photorezeptor kann zum zum Hochdurchsatzscreening von pH-regulierten Membranproteinen ausgebildet sein.

Der biologische Photorezeptor kann zum Hochdurchsatzscreening von spannungsabhängigen Membranproteinen.

Der lichtgesteuerte Ionenkanal kann in Kombination mit einem lichtgesteuerten aktiven lonentransportsystem ausgebildet oder vorgesehen sein.

Offenbart wird auch die Verwendung des biologischen Photorezeptors zur Herstellung eines Mittels zur Behandlung eines Säugetiers, welches an einer teilweisen oder vollständigen Erblindung leidet oder an einer Krankheit erkrankt ist, die eine teilweise oder vollständige Erblindung als Symptom oder zur Folge hat.

Offenbart wird ferner auch die Verwendung des biologischen Photorezeptors zur Herstellung eines Mittels zur Behandlung eines Säugetiers, welches an einer neurologischen Krankheit leidet.

Der biologische Photorezeptor kann dabei als pharmakologisch wirksame Substanz oder als Teil davon eingesetzt werden.

Das Mittel kann ein System, ein Gerät, ein Kit und/oder ein Medikament sein oder aufweisen und der biologische Photorezeptor kann ein wirksamer Teil davon sein.

Das Mittel kann zur Behandlung eines Menschen dienen oder verwendet werden.

## Patentansprüche

1. Biologischer Photorezeptor in der Form eines direkt lichtgesteuerten Ionenkanals, zur Veränderung der lonenleitfähigkeit einer Membran mit Hilfe von Licht,
- welcher ein Apoprotein und ein kovalent an das Apoprotein gebundenes lichtsensitives Polyen, welches mit dem Apoprotein wechselwirkt und als lichtempfindliches Tor fungiert, aufweist,
- wobei das Apoprotein mindestens die Aminosäuren 24 bis 268 des Channelopsin2 (CHOP-2) gemäss der in Fig. 1 B gezeigten Aminosäuresequenz, die an Position 134 durch einen Austausch mit einer anderen Aminosäure verändert sind, umfasst.

2. Biologischer Photorezeptor nach Anspruch 1, wobei die Aminosäure Histidin an Position 134 des Channelopsin2 (CHOP-2) durch Arginin ersetzt ist.

3. Biologischer Photorezeptor nach einem der vorangehenden Ansprüche, wobei das lichtsensitive Polyen ein Retinal oder Retinalderivat ist.

4. Biologischer Photorezeptor nach Anspruch 3, wobei das Retinalderivat aus der folgenden Gruppe ausgewählt ist: 3,4-Dehydroretinal, 13-Ethylretinal, 9-dm-Retinal, 3-Hydroxyretinal, 4-Hydroxyretinal, Naphthylretinal; 3,7,11-Trimethyl-dodeca-2,4,6,8,10-pentaenal; 3,7-Dimethyl-deca-2,4,6,8-tetraenal; 3,7-Dimethyl-octa-2,4,6-trienal; sowie 6-7- oder 8-9- oder 10-11-Rotations-blockierte Retinale.

5. DNA, kodierend den biologischen Photorezeptor nach einem der vorangehenden Ansprüche 1 bis 4.

6. DNA nach Anspruch 5, wobei die DNA in einem Expressionsvektor, insbesondere einem Plasmid, Cosmid oder viralen Vektor vorliegt.

7. DNA nach Anspruch 6, wobei die DNA mit weiteren regulatorischen Sequenzen verknüpft ist.

8. Biologischer Photorezeptor in der Form eines direkt lichtgesteuerten Ionenkanals, zur Veränderung der Ionenleitfähigkeit einer Membran mit Hilfe von Licht,
- welcher ein Apoprotein und ein kovalent an das Apoprotein gebundenes lichtsensitives Polyen, welchesmit dem Apoprotein wechselwirkt und als lichtempfindliches Tor fungiert, aufweist, wobei das lichtsensitive Polyen ein Retinal oder Retinalderivat ist,
- wobei das Apoprotein mindestens die Aminosäuren 24 bis 268 des Channelopsin 2 (CHOP-2) gemäss der in Fig. 1 B gezeigten Aminosäuresequenz umfasst, oder wobei das Apoprotein mindestens die Aminosäuren 24 bis 268 des Channelopsin2 (CHOP-2) gemäss der in Fig. 1 B gezeigten Aminosäuresequenz , die an einer Position durch einen Austausch einer Aminosäure verändert sind, umfasst.

9. Biologischer Photorezeptor nach Anspruch 8, wobei die Aminosäure Histidin an Position 134 des Channelopsin2 (CHOP-2) durch eine andere Aminosäure ersetzt ist.

10. Biologischer Photorezeptor nach Anspruch 9, wobei die Aminosäure Histidin an Position 134 des Channelopsin2 (CHOP-2) durch Arginin ersetzt ist.

11. Biologischer Photorezeptor nach Anspruch 8, wobei das Retinalderivat aus der folgenden Gruppe ausgewählt ist: 3,4-Dehydroretinal, 13-Ethylretinal, 9-dm-Retinal, 3-Hydroxyretinal, 4-Hydroxyretinal, Naphthylretinal; 3,7,11-Trimethyl-dodeca-2,4,6,8,10-pentaenal; 3,7-Dimethyl-deca-2,4,6,8-tetraenal; 3,7-Dimethyl-octa-2,4,6-trienal; sowie 6-7- oder 8-9- oder 10-11-Rotations-blockierte Retinale.

## Claims

1. A biological photoreceptor in form of a directly light-controlled ion channel, for the alteration of the ion conductivity of a membrane with the aid of light,
- which has an apoprotein and a light-sensitive polyene covalently bound to the apoprotein, which interacts with the apoprotein and functions as a light-sensitive gate,
- wherein the apoprotein comprises at least amino acids 24 to 268 of the Channelopsin2 (CHOP-2) according to the amino acid sequence shown in Fig. 1 B, which is altered in position 134 by a replacement by another amino acid.

2. The biological photoreceptor of claim 1, wherein the amino acid histidine at position 134 of the Channelopsin2 (CHOP-2) is replaced by arginine.

3. The biological photoreceptor of any one of the preceding claims, wherein the light-sensitive polyene is a retinal or retinal derivative.

4. The biological photoreceptor of claim 3, wherein the retinal derivative is selected from the following group: 3,4-dehydroretinal, 13-ethylretinal, 9-dm-retinal, 3-hydroxyretinal, 4-hydroxyretinal, naphthyl retinal; 3,7,11-trimethyl-dodeca-2,4,6,8,10-pentaenal; 3,7-dimethyl-deca-2,4,6,8-tetraenal; 3,7-dimethyl-octa-2,4,6-trienal; as well as 6-7- or 8-9- or 10-11 rotation-blocked retinals.

5. A DNA, encoding the biological photoreceptor according to one of the preceding claims 1 to 4.

6. The DNA of claim 5, wherein the DNA is present in an expression vector, in particular in a plasmid, cosmid or viral vector.

7. The DNA of claim 6, wherein the DNA is linked with further regulatory sequences.

8. A biological photoreceptor in form of a directly light-controlled ion channel, for the alteration of the ion conductivity of a membrane with the aid of light,
- which has an apoprotein and a light-sensitive polyene covalently bound to the apoprotein, which interacts with the apoprotein and functions as a light-sensitive gate, wherein the light-sensitive polyene is a retinal or retinal derivative,
- wherein the apoprotein comprises at least amino acids 24 to 268 of the Channelopsin2 (CHOP-2) according to the amino acid sequence shown in Fig. 1 B, or wherein the apoprotein comprises at least amino acids 24 to 268 of the Channelopsin2 (CHOP-2) according to the amino acid sequence shown in Fig. 1 B, which are altered at a position by a replacement by another amino acid.

9. The biological photoreceptor of claim 8, wherein the amino acid histidine at position 134 of the Channelopsin2 (CHOP-2) is replaced by another amino acid.

10. The biological photoreceptor of claim 9, wherein the amino acid histidine at position 134 of the Channelopsin2 (CHOP-2) is replaced by arginine.

11. The biological photoreceptor of claim 8, wherein the retinal derivative is selected from the following group: 3,4-dehydroretinal, 13-ethylretinal, 9-dm-retinal, 3-hydroxyretinal, 4-hydroxyretinal, naphthylretinal; 3,7,11-trimethyl-dodeca-2,4,6,8,10-pentaenal; 3,7-dimethyl-deca-2,4,6,8-tetraenal; 3,7-dimethyl-octa-2,4,6-trienal; as well as 6-7- or 8-9- or 10-11 rotation-btocked retinals.

## Revendications

1. Photorécepteur biologique sous la forme d'un canal ionique commandé directement par la lumière, pour faire varier la conductivité ionique d'une membrane à l'aide de la lumière,
- lequel présente une apoprotéine et un polyène photosensible lié à l'apoprotéine par covalence, lequel interagit avec l'apoprotéine et fait office de porte photosensible,
- dans lequel l'apoprotéine comprend au moins les acides aminés 24 à 268 de l'opsine de canal 2 (CHOP-2) selon la séquence d'acides aminés représentée sur la figure 1 B qui est modifiée à la position 134 par un échange avec un autre acide aminé.

2. Photorécepteur biologique selon la revendication 1, dans lequel l'acide aminé histidine en position 134 de l'opsine de canal 2 (CHOP-2) est remplacé par l'arginine.

3. Photorécepteur biologique selon une des revendications précédentes, dans lequel le polyène photosensible est un rétinal ou un dérivé de rétinal.

4. Photorécepteur biologique selon la revendication 3, dans lequel le dérivé de rétinal est sélectionné dans le groupe suivant : 3,4-déshydrorétinal, 13-éthylrétinal, 9-dm-rétinal, 3-hydroxyrétinal, 4-hydroxyrétinal, naphtylrétinal ; 3,7,11-triméthyldodéca-2,4,6,8,10-pentaénal ; 3,7-diméthyldéca-2,4,6,8-tétraénal ; 3,7-diméthylocta-2,4,6-triénal ; et 6-7- ou 8-9- ou 10-11-rétinal à rotation bloquée.

5. ADN codant le photorécepteur biologique selon une des revendications précédentes 1 à 4.

6. ADN selon la revendication 5, lequel ADN est présent dans un vecteur d'expression, en particulier un plasmide, un cosmide ou un vecteur viral.

7. ADN selon la revendication 6, lequel ADN est lié à d'autres séquences régulatrices.

8. Photorécepteur biologique sous la forme d'un canal ionique commandé directement par la lumière, pour faire varier la conductivité ionique d'une membrane à l'aide de la lumière,
- lequel présente une apoprotéine et un polyène photosensible lié à l'apoprotéine par covalence, lequel interagit avec l'apoprotéine et fait office de porte photosensible, le polyène photosensible étant un rétinal ou un dérivé de rétinal,
- dans lequel l'apoprotéine comprend au moins les acides aminés 24 à 268 de l'opsine de canal 2 (CHOP-2) selon la séquence d'acides aminés représentée sur la figure 1 B, ou dans lequel l'apoprotéine comprend au moins les acides aminés 24 à 268 de l'opsine de canal 2 (CHOP-2) selon la séquence d'acides aminés représentée sur la figure 1 B qui est modifiée à une position par un échange d'un acide aminé.

9. Photorécepteur biologique selon la revendication 8, dans lequel l'acide aminé histidine en position 134 de l'opsine de canal 2 (CHOP-2) est remplacé par un autre acide aminé.

10. Photorécepteur biologique selon la revendication 9, dans lequel l'acide aminé histidine en position 134 de l'opsine de canal 2 (CHOP-2) est remplacé par l'arginine.

11. Photorécepteur biologique selon la revendication 8, dans lequel le dérivé de rétinal est sélectionné dans le groupe suivant : 3,4-déshydrorétinal, 13-éthylrétinal, 9-dm-rétinal, 3-hydroxyrétinal, 4-hydroxyrétinal, naphtylrétinal ; 3,7,11-triméthyl-dodéca-2,4,6,8,10-pentaénal ; 3,7-diméthyldéca-2,4,6,8-tétraénal ; 3,7-diméthyl-octa-2,4,6-triénal ; et 6-7- ou 8-9- ou 10-11-rétinal à rotation bloquée.


### SEQUENZPROTOKOLL

<110> Peter, Hegemann und Max-Planck-Gesellschaft zur Förderung der Wissenschaften e. v.
<120> Verwendung von biologischen Photorezeptoren als direkt
   lichtgesteuerte Ionenkanäle
<130> P33838-019
<160> 3
<170> PatentIn version 3.1
<210> 1
   <211> 712
   <212> PRT
   <213> Chlamydomonas reinhardtii
<220>
   <223> Aminosäuresequenz von CHOP-1 (AF385748) aus Chlamydomonas reinhardtii
<400> 1
   Met Ser Arg Arg Pro Trp Leu Leu Ala Leu Ala Leu Ala Val Ala Leu
   1               5                   10                  15
   Ala Ala Gly Ser Ala Gly Ala Ser Thr Gly Ser Asp Ala Thr Val Pro
               20                  25                  30
   Val Ala Thr Gln Asp Gly Pro Asp Tyr Val Phe His Arg Ala His Glu
           35                  40                  45
   Arg Met Leu Phe Gln Thr Ser Tyr Thr Leu Glu Asn Asn Gly Ser Val
       50                  55                  60
   Ile Cys Ile Pro Asn Asn Gly Gln Cys Phe Cys Leu Ala Trp Leu Lys
   65 70 75 80
   Ser Asn Gly Thr Asn Ala Glu Lys Leu Ala Ala Asn Ile Leu Gln Trp
   85 90 95
   Ile Thr Phe Ala Leu Ser Ala Leu Cys Leu Met Phe Tyr Gly Tyr Gln
   100 105 110
   Thr Trp Lys Ser Thr Cys Gly Trp Glu Glu Ile Tyr Val Ala Thr Ile
   115 120 125
   Glu Met Ile Lys Phe Ile Ile Glu Tyr Phe His Glu Phe Asp Glu Pro
   130 135 140
   Ala Val Ile Tyr Ser Ser Asn Gly Asn Lys Thr Val Trp Leu Arg Tyr
   145 150 155 160
   Ala Glu Trp Leu Leu Thr Cys Pro Val Ile Leu Ile His Leu Ser Asn
   165 170 175
   Leu Thr Gly Leu Ala Asn Asp Tyr Asn Lys Arg Thr Met Gly Leu Leu
   180 185 190
   Val Ser Asp Ile Gly Thr Ile Val Trp Gly Thr Thr Ala Ala Leu Ser
   195 200 205
   Lys Gly Tyr Val Arg Val Ile Phe Phe Leu Met Gly Leu Cys Tyr Gly
   210 215 220
   Ile Tyr Thr Phe Phe Asn Ala Ala Lys Val Tyr Ile Glu Ala Tyr His
   225 230 235 240
   Thr Val Pro Lys Gly Ile Cys Arg Asp Leu Val Arg Tyr Leu Ala Trp
   245 250 255
   Leu Tyr Phe Cys Ser Trp Ala Met Phe Pro Val Leu Phe Leu Leu Gly
   260 265 270
   Pro Glu Gly Phe Gly His Ile Asn Gln Phe Asn Ser Ala Ile Ala His
   275 280 285
   Ala Ile Leu Asp Leu Ala Ser Lys Asn Ala Trp Ser Met Met Gly His
   290 295 300
   Phe Leu Arg Val Lys Ile His Glu His Ile Leu Leu Tyr Gly Asp Ile
   305 310 315 320
   Arg Lys Lys Gln Lys Val Asn Val Ala Gly Gln Glu Met Glu Val Glu
   325 330 335
   Thr Met Val His Glu Glu Asp Asp Glu Thr Gln Lys Val Pro Thr Ala
   340 345 350
   Lys Tyr Ala Asn Arg Asp Ser Phe Ile Ile Met Arg Asp Arg Leu Lys
   355 360 365
   Glu Lys Gly Phe Glu Thr Arg Ala Ser Leu Asp Gly Asp Pro Asn Gly
   370 375 380
   Asp Ala Glu Ala Asn Ala Ala Ala Gly Gly Lys Pro Gly Met Glu Met
   385 390 395 400
   Gly Lys Met Thr Gly Met Gly Met Gly Met Gly Ala Gly Met Gly Met
   405 410 415
   Ala Thr Ile Asp Ser Gly Arg Val Ile Leu Ala Val Pro Asp Ile Ser
   420 425 430
   Met Val Asp Phe Phe Arg Glu Gln Phe Ala Arg Leu Pro Val Pro Tyr
   435 440 445
   Glu Leu Val Pro Ala Leu Gly Ala Glu Asn Thr Leu Gln Leu Val Gln
   450 455 460
   Gln Ala Gln Ser Leu Gly Gly Cys Asp Phe Val Leu Met His Pro Glu
   465 470 475 480
   Phe Leu Arg Asp Arg Ser Pro Thr Gly Leu Leu Pro Arg Leu Lys Met
   485 490 495
   Gly Gly Gln Arg Ala Ala Ala Phe Gly Trp Ala Ala Ile Gly Pro Met
   500 505 510
   Arg Asp Leu Ile Glu Gly Ser Gly Val Asp Gly Trp Leu Glu Gly Pro
   515 520 525
   Ser Phe Gly Ala Gly Ile Asn Gln Gln Ala Leu Val Ala Leu Ile Asn
   530 535 540
   Arg Met Gln Gln Ala Lys Lys Met Gly Met Met Gly Gly Met Gly Met
   545                 550                 555                 560
   Gly Met Gly Gly Gly Met Gly Met Gly Met Gly Met Gly Met Gly Met
                   565                 570                 575
   Ala Pro Ser Met Asn Ala Gly Met Thr Gly Gly Met Gly Gly Ala Ser
               580                 585                 590
   Met Gly Gly Ala Val Met Gly Met Gly Met Gly Met Gln Pro Met Gln
           595                 600                 605
   Gln Ala Met Pro Ala Met Ser Pro Met Met Thr Gln Gln Pro Ser Met
       610                 615                 620
   Met Ser Gln Pro Ser Ala Met Ser Ala Gly Gly Ala Met Gln Ala Met
   625                 630                 635                 640
   Gly Gly Val Met Pro Ser Pro Ala Pro Gly Gly Arg Val Gly Thr Asn
                   645                 650                 655
   Pro Leu Phe Gly Ser Ala Pro Ser Pro Leu Ser Ser Gln Pro Gly Ile
               660                 665                 670
   Ser Pro Gly Met Ala Thr Pro Pro Ala Ala Thr Ala Ala Pro Ala Ala
           675                 680                 685
   Gly Gly Ser Glu Ala Glu Met Leu Gln Gln Leu Met Ser Glu Ile Asn
       690                 695                 700
   Arg Leu Lys Asn Glu Leu Gly Glu
   705                 710
<210> 2
   <211> 737
   <212> PRT
   <213> Chlamydomonas reinhardtii
<220>
   <223> Aminosäuresequenz von CHOP-2 (AF461397) aus Chlamydomonas reinhardtii
<400> 2
   Met Asp Tyr Gly Gly Ala Leu Ser Ala Val Gly Arg Glu Leu Leu Phe
   1               5                   10                  15
   Val Thr Asn Pro Val Val Val Asn Gly Ser Val Leu Val Pro Glu Asp
               20                  25                  30
   Gln Cys Tyr Cys Ala Gly Trp Ile Glu Ser Arg Gly Thr Asn Gly Ala
           35                  40                  45
   Gln Thr Ala Ser Asn Val Leu Gln Trp Leu Ala Ala Gly Phe Ser Ile
       50                  55                  60
   Leu Leu Leu Met Phe Tyr Ala Tyr Gln Thr Trp Lys Ser Thr Cys Gly
   65                  70                  75                  80
   Trp Glu Glu Ile Tyr Val Cys Ala Ile Glu Met Val Lys Val Ile Leu
                   85                  90                  95
   Glu Phe Phe Phe Glu Phe Lys Asn Pro Ser Met Leu Tyr Leu Ala Thr
               100                 105                 110
   Gly His Arg Val Gln Trp Leu Arg Tyr Ala Glu Trp Leu Leu Thr Cys
           115                 120                 125
   Pro Val Ile Leu Ile His Leu Ser Asn Leu Thr Gly Leu Ser Asn Asp
       130                 135                 140
   Tyr Ser Arg Arg Thr Met Gly Leu Leu Val Ser Asp Ile Gly Thr Ile
   145                 150                 155                 160
   Val Trp Gly Ala Thr Ser Ala Met Ala Thr Gly Tyr Val Lys Val Ile
                   165                 170                 175
   Phe Phe Cys Leu Gly Leu Cys Tyr Gly Ala Asn Thr Phe Phe His Ala
               180                 185                 190
   Ala Lys Ala Tyr Ile Glu Gly Tyr His Thr Val Pro Lys Gly Arg Cys
           195                 200                 205
   Arg Gln Val Val Thr Gly Met Ala Trp Leu Phe Phe Val Ser Trp Gly
       210                 215                 220
   Met Phe Pro Ile Leu Phe Ile Leu Gly Pro Glu Gly Phe Gly Val Leu
   225                 230                 235                 240
   Ser Val Tyr Gly Ser Thr Val Gly His Thr Ile Ile Asp Leu Met Ser
                   245                 250                 255
   Lys Asn Cys Trp Gly Leu Leu Gly His Tyr Leu Arg Val Leu Ile His
               260                 265                 270
   Glu His Ile Leu Ile His Gly Asp Ile Arg Lys Thr Thr Lys Leu Asn
           275                 280                 285
   Ile Gly Gly Thr Glu Ile Glu Val Glu Thr Leu Val Glu Asp Glu Ala
       290                 295                 300
   Glu Ala Gly Ala Val Asn Lys Gly Thr Gly Lys Tyr Ala Ser Arg Glu
   305                 310                 315                 320
   Ser Phe Leu Val Met Arg Asp Lys Met Lys Glu Lys Gly Ile Asp Val
                   325                 330                 335
   Arg Ala Ser Leu Asp Asn Ser Lys Glu Val Glu Gln Glu Gln Ala Ala
               340                 345                 350
   Arg Ala Ala Met Met Met Met Asn Gly Asn Gly Met Gly Met Gly Met
           355                 360                 365
   Gly Met Asn Gly Met Asn Gly Met Gly Gly Met Asn Gly Met Ala Gly
       370                 375                 380
   Gly Ala Lys Pro Gly Leu Glu Leu Thr Pro Gln Leu Gln Pro Gly Arg
   385                 390                 395                 400
   Val Ile Leu Ala Val Pro Asp Ile Ser Met Val Asp Phe Phe Arg Glu
                   405                 410                 415
   Gln Phe Ala Gln Leu Ser Val Thr Tyr Glu Leu Val Pro Ala Leu Gly
               420                 425                 430
   Ala Asp Asn Thr Leu Ala Leu Val Thr Gln Ala Gln Asn Leu Gly Gly
           435                 440                 445
   Val Asp Phe Val Leu Ile His Pro Glu Phe Leu Arg Asp Arg Ser Ser
       450                 455                 460
   Thr Ser Ile Leu Ser Arg Leu Arg Gly Ala Gly Gln Arg Val Ala Ala
   465 470 475 480
   Phe Gly Trp Ala Gln Leu Gly Pro Met Arg Asp Leu Ile Glu Ser Ala
   485 490 495
   Asn Leu Asp Gly Trp Leu Glu Gly Pro Ser Phe Gly Gln Gly Ile Leu
   500 505 510
   Pro Ala His Ile Val Ala Leu Val Ala Lys Met Gln Gln Met Arg Lys
   515 520 525
   Met Gln Gln Met Gln Gln Ile Gly Met Met Thr Gly Gly Met Asn Gly
   530 535 540
   Met Gly Gly Gly Met Gly Gly Gly Met Asn Gly Met Gly Gly Gly Asn
   545 550 555 560
   Gly Met Asn Asn Met Gly Asn Gly Met Gly Gly Gly Met Gly Asn Gly
   565 570 575
   Met Gly Gly Asn Gly Met Asn Gly Met Gly Gly Gly Asn Gly Met Asn
   580 585 590
   Asn Met Gly Gly Asn Gly Met Ala Gly Asn Gly Met Gly Gly Gly Met
   595 600 605
   Gly Gly Asn Gly Met Gly Gly Ser Met Asn Gly Met Ser Ser Gly Val
   610 615 620
   Val Ala Asn Val Thr Pro Ser Ala Ala Gly Gly Met Gly Gly Met Met
   625 630 635 640
   Asn Gly Gly Met Ala Ala Pro Gln Ser Pro Gly Met Asn Gly Gly Arg
   645 650 655
   Leu Gly Thr Asn Pro Leu Phe Asn Ala Ala Pro Ser Pro Leu Ser Ser
   660 665 670
   Gln Leu Gly Ala Glu Ala Gly Met Gly Ser Met Gly Gly Met Gly Gly
   675 680 685
   Met Ser Gly Met Gly Gly Met Gly Gly Met Gly Gly Met Gly Gly Ala
   690 695 700
   Gly Ala Ala Thr Thr Gln Ala Ala Gly Gly Asn Ala Glu Ala Glu Met
   705 710 715 720
   Leu Gln Asn Leu Met Asn Glu Ile Asn Arg Leu Lys Arg Glu Leu Gly
   725 730 735
   Glu
<210> 3
   <211> 259
   <212> PRT
   <213> Halobacterium salinarum
<220>
   <223> Aminosäuresequenz von Bakteriorhodopsin aus Halobakterium salinarum
<400> 3
   Met Leu Pro Thr Ala Val Glu Gly Val Ser Gln Ala Gln Ile Thr Gly
   1 5 10 15
   Arg Pro Glu Trp Ile Trp Leu Ala Leu Gly Thr Ala Leu Met Gly Leu
   20 25 30
   Gly Thr Leu Tyr Phe Leu Val Lys Gly Met Gly Val Ser Asp Pro Asp
   35 40 45
   Ala Lys Lys Phe Tyr Ala Ile Thr Thr Leu Val Pro Ala Ile Ala Phe
   50 55 60
   Thr Met Tyr Leu Ser Met Leu Leu Gly Tyr Gly Leu Thr Met Val Pro
   65 70 75 80
   Phe Gly Gly Glu Gln Asn Pro Ile Tyr Trp Ala Arg Tyr Ala Asp Trp
   85 90 95
   Leu Phe Thr Thr Pro Leu Leu Leu Leu Asp Leu Ala Leu Leu Val Asp
   100 105 110
   Ala Asp Gln Gly Thr Ile Leu Ala Leu Val Gly Ala Asp Gly Ile Met
               115                 120                 125
   Ile Gly Thr Gly Leu Val Gly Ala Leu Thr Lys Val Tyr Ser Tyr Arg
       130                 135                 140
   Phe Val Trp Trp Ala Ile Ser Thr Ala Ala Met Leu Tyr Ile Leu Tyr
   145                 150                 155                 160
   Val Leu Phe Phe Gly Phe Thr Ser Lys Ala Glu Ser Met Arg Pro Glu
                   165                 170                 175
   Val Ala Ser Thr Phe Lys Val Leu Arg Asn Val Thr Val Val Leu Trp
               180                 185                 190
   Ser Ala Tyr Pro Val Val Trp Leu Ile Gly Ser Glu Gly Ala Gly Ile
           195                 200                 205
   Val Pro Leu Asn Ile Glu Thr Leu Leu Phe Met Val Leu Asp Val Ser
       210                 215                 220
   Ala Lys Val Gly Phe Gly Leu Ile Leu Leu Arg Ser Arg Ala Ile Phe
   225                 230                 235                 240
   Gly Glu Ala Glu Ala Pro Glu Pro Ser Ala Gly Asp Gly Ala Ala Ala
                   245                 250                 255
   Thr Ser Asp